# EUROPEAN PATENT APPLICATION

(11) **EP 4 685 230 A1**
(43) Date of publication of application: **28.01.2026**
(21) Application number: 24774924.5
(22) Date of filing: 19.03.2024
(51) Int. Cl.: C12N 5/0797, A61K 35/30, A61P 25/28, C12N 5/10, C12Q 1/02

(54) **METHOD FOR PRODUCING NEURAL ORGANOIDS AND USE OF SAME**

(30) Priority: 20.03.2023 JP 2023044570
(71) Applicant: KYOTO UNIVERSITY, Kyoto 606-8501 (JP); Tosoh Corporation, Yamaguchi 746-8501 (JP)
(72) Inventor: TAKAHASHI, Jun, Kyoto-shi, Kyoto 606-8501 (JP); SAMATA, Bumpei, Kyoto-shi, Kyoto 606-8501 (JP); DOI, Daisuke, Kyoto-shi, Kyoto 606-8501 (JP); IMAIZUMI, Yu, Yokkaichi-shi, Mie 510-8540 (JP); KUNO, Goshi, Yokkaichi-shi, Mie 510-8540 (JP)
(74) Representative: Novagraaf International SA
(86) International application number: PCT/JP2024/010698
(87) International publication number: WO 2024/195789

(57) **Abstract**

The present invention provides a method for producing neural organoids, the method comprising: (1) a step for performing adhesive culturing of pluripotent stem cells in a culturing vessel that has multiple independent cell-adhesive regions and that allows cells adhered to the respective regions to exist in the same medium, and forming cell populations including neural stem cells; and (2) a step for detaching the cell populations including the neural stem cells formed in step (1) and performing suspension culturing of the same.

## Description

### TECHNICAL FIELD

The present invention relates to a method for producing a neural organoid using a culture vessel that has a plurality of independent cell attachable regions and is configured to have cells that will attach to the regions to be present in the same culture medium. The present invention also relates to a transplantation therapy agent containing the neural organoid, and a method for screening a therapeutic or prophylactic medicine for a neurodegenerative disease using the neural organoid.

### BACKGROUND ART

There is increasing interest in drug development for a neurodegenerative disease such as amyotrophic lateral sclerosis (ALS) using neural stem cells or a neural organoid obtained by inducing differentiation of pluripotent stem cells (e.g., induced pluripotent stem cells (iPS cells)). For example, it is expected that a disease mechanism that has been difficult to clarify hitherto will be clarified, and drug development research and regenerative medicine will be developed, by putting iPS cells derived from patients suffering from diseases.

Conventionally, as a method for inducing differentiation into a neural organoid (e.g., cerebral organoid), culture in a 96-well plate has been mainstream (Patent Literature 1, Non-Patent Literature 1 and 2). However, the wells of a 96-well plate are independent of one another, and in many cases, the morphological differences and the like (e.g., the size, the expression of a marker gene, and the like) of individual cerebral organoids are extremely large throughout the induction period. There are products, such as EZSPHERE, having a plurality of projections and depressions inside a dish for the purpose of producing a plurality of organoids in the same space, but such products are designed based on the premise that cells are uniformly placed through gravity-drop when a cell suspension is seeded, and as a result, the problem of occurrence of a difference between individual cerebral organoids remains unsolved. Also, there is a problem in that adjacent organoids join together by being slightly shaken due to a culturing operation and the like.

The inventors of the present invention have been hitherto conducting research on a method for inducting differentiation of pluripotent stem cells into neural stem cells or nerve cells using a technique for a micropatterning method (Patent Literature 2). However, a method for producing a neural organoid derived from pluripotent stem cells with stable quality in a large amount at low cost has not been known yet.

### Citation List

### Patent Literature

Patent Literature 1: WO 2015/076388
Patent Literature 2: JP 2021-158960A

### Non-Patent Literature

Non-Patent Literature 1: Sakaguchi H, et al., Stem Cell Reports, 10; 13(3): 458-473 (2019)
Non-Patent Literature 2: Kadoshima T, et al., Proc Natl Acad Sci U S A. 110(50): 20284-20289 (2013)

### SUMMARY OF INVENTION

### Technical Problem

Accordingly, it is an object of the present invention to provide a method for producing a neural organoid using a culture vessel that has a plurality of independent cell attachable regions and is configured to have cells that will attach to the regions to be present in the same culture medium, in order to realize production of a neural organoid derived from pluripotent stem cells with stable quality in a large amount at low cost. Also, it is another object thereof to provide a transplantation therapy agent containing a neural organoid obtained using the production method of the present invention, and a method for screening a therapeutic or prophylactic medicine for a neurodegenerative disease using the neural organoid.

### Solution to Problem

The inventors of the present invention examined use of a technique for a micropatterning method from the viewpoint of improving the quality stability and production efficiency of a neural organoid, and came up with an idea of using a culture vessel that has previously determined cell attachable regions and is configured to have cells that will attach to the regions to be present in the same culture medium. Surprisingly, it was found that neural organoids can be produced from uniform cells by using the culture vessel to allow the initial induction stages of neural organoids to simultaneously occur. It was also found that since the cell attachable regions have been determined in advance in the culture vessel, it is possible to prevent, for example, the adjacent cells from attaching to each other due to the operation such as replacement of the culture medium. As a result of intensive studies, it was found that using the culture vessel makes it possible to improve quality stabilization and production efficiency of the production of a neural organoid derived from pluripotent stem cells. As a result of conducting further studies based on these findings, the inventors have achieved the production method of the present invention for producing a neural organoid using a culture vessel that has a plurality of independent cell attachable regions and is configured to have cells that will attach to the regions to be present in the same culture medium.

That is to say, the present invention is as follows.
[1-1] A method for producing a neural organoid, including:
   (1) a step of forming a cell population containing neural stem cells through adherent culture of pluripotent stem cells in a culture vessel that has a plurality of independent cell attachable regions and is configured to have cells that will attach to the regions to be present in the same culture medium; and
   (2) a step of detaching the cell population containing neural stem cells formed in the step (1) and conducting suspension culture of the cell population.
[1-2] The method according to [1-1], wherein the suspension culture of the step (2) includes a step of conducting rotation culture, culture under a turbulent condition, culture under a microgravity environment, or hanging-drop culture.
[2-1] The method according to [1-1] or [1-2], wherein the suspension culture of the step (2) includes a step of conducting rotation culture.
[2-2] The method according to [2-1], wherein the suspension culture of the step (2) also includes a step of conducting static culture.
[3] The method according to any one of [1-1] to [2-2], wherein the neural organoid is selected from the group consisting of a brain organoid, a cerebral organoid, a cerebellar organoid, a spinal organoid, a mesencephalic organoid, a choroid plexus organoid, a hippocampal organoid, a hypothalamic organoid, an anterior pituitary organoid, and a basal ganglia organoid.
[4] The method according to any one of [1-1] to [3], wherein the neural organoid is a cerebral organoid.
[5] The method according to any one of [1-1] to [4], wherein the step (1) is conducted for at least 6 days.
[6] The method according to any one of [1-1] to [5], wherein the cell attachable regions contain extracellular matrix.
[7] The method according to any one of [1-1] to [6], wherein the cell attachable regions contain a temperature-responsive polymer.
[8] The method according to any one of [1-1] to [7], wherein the number of the cell attachable regions is five or more.
[9] The method according to any one of [1-1] to [8], wherein the pluripotent stem cells are induced pluripotent stem cells.
[10] The method according to [9], wherein the induced pluripotent stem cells are derived from a patient suffering from a neurodegenerative disease.
[11] A neural organoid produced using the method according to any one of [1-1] to [10].
[12-1] A transplantation therapy agent containing the neural organoid according to [11] or cells included in the neural organoid.
[12-2] The agent according to [12-1] for treating a disease or damage caused by a disorder of a telencephalon, a diencephalon, a mesencephalon, a metencephalon, or a spinal cord.
[12-3] The agent according to [12-2], wherein the disease or damage caused by a disorder of a telencephalon, a diencephalon, a mesencephalon, a metencephalon, or a spinal cord is selected from the group consisting of the Parkinsons's disease, the Huntington's chorea, the Alzheimer's disease, a cerebrovascular disorder, epilepsy, a traumatic brain injury, a motor neuron disease, a neurodegenerative disease, and damage after a brain surgery.
[13] A method for screening a therapeutic or prophylactic medicine for a neurodegenerative disease, including:
   (1) a step of culturing the neural organoid according to [11] or cells included in the neural organoid in the presence or absence of a test substance;
   (2) a step of evaluating a degree of neurodegeneration of the neural organoid or the cells included in the neural organoid; and
   (3) a step of selecting the test substance as a candidate substance of a therapeutic or prophylactic medicine for a neurodegenerative disease when progression of the neurodegeneration of the neural organoid or the cells included in the neural organoid is more suppressed in the presence of the test substance than in the absence of the test substance in the step (2).
[14-1] A method for treating a disease or damage caused by a disorder of a telencephalon, a diencephalon, a mesencephalon, a metencephalon, or a spinal cord in a mammal, wherein an effective amount of the neural organoid according to [11] or the agent according to any one of [12-1] to [12-3] is administered to the mammal.
[14-2] The treatment method according to [14-1], wherein the disease or damage caused by a disorder of a telencephalon, a diencephalon, a mesencephalon, a metencephalon, or a spinal cord is selected from the group consisting of the Parkinsons's disease, the Huntington's chorea, the Alzheimer's disease, a cerebrovascular disorder, epilepsy, a traumatic brain injury, a motor neuron disease, a neurodegenerative disease, and damage after a brain surgery.
[15] The neural organoid according to [11] for use to treat a disease or damage caused by a disorder of a telencephalon, a diencephalon, a mesencephalon, a metencephalon, or a spinal cord.
[16] Use of the neural organoid according to [11] to produce a therapeutic agent for a disease or damage caused by a disorder of a telencephalon, a diencephalon, a mesencephalon, a metencephalon, or a spinal cord.

### Advantageous Effects of Invention

With the present invention, it is possible to provide a method for producing a neural organoid using a culture vessel that has a plurality of independent cell attachable regions and is configured to have cells that will attach to the regions to be present in the same culture medium, and the method can be used to produce a neural organoid with stable quality in a large amount at low cost. Since the cell attachable regions are determined in advance in the culture vessel used in the present invention, it is possible to prevent, for example, the adjacent cells from attaching to each other due to the operation such as replacement of the culture medium. Therefore, it is possible to produce neural organoids with stable quality in a large amount through a single operation. Also, the neural organoid produced using the production method of the present invention has stable (equal) quality, and therefore, it is expected that a mechanism of a disease (particularly a neurodegenerative disease) that has been difficult to clarify hitherto will be clarified, and drug development research, regenerative medicine, and the like will be developed, by using the transplantation therapy agent containing the neural organoid, or the method for screening a therapeutic or prophylactic medicine for a neurodegenerative disease using the neural organoid.

### Brief Description of Drawings

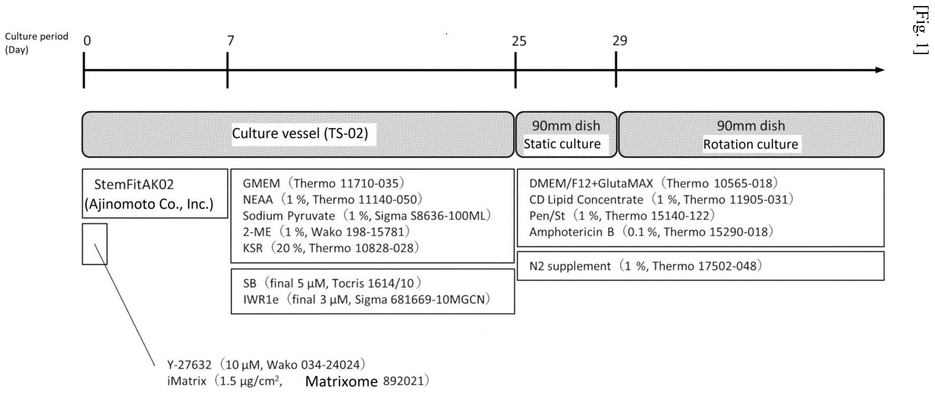

[FIG. 1] FIG. 1 shows the outline of a method for culturing a human cerebral cortex organoid. Cells were seeded on a TS-02 substrate at a cell density of 15,000 cells/cm² using a StemFit (AK02) culture medium, and this day was taken as Day 0 of the culture. The seeded cells became confluent on Day 7 of the culture. Thereafter, induction was conducted under conditions where the culture medium supplemented with KSR (knockout serum replacement), SB431542, and IWR-1-endo between Days 7 and 25 of the culture. The cells were detached on Day 25, and were transferred onto a 90-mm dish. Then, the cells were cultured for 4 days in the culture medium supplemented with GlutaMAX, CD Lipid concentrate, Penicillin/Streptomycin, Amphotericin B (0.1%), and N2-supplement (1%). Rotation culture was conducted on Day 29 of the culture onward.

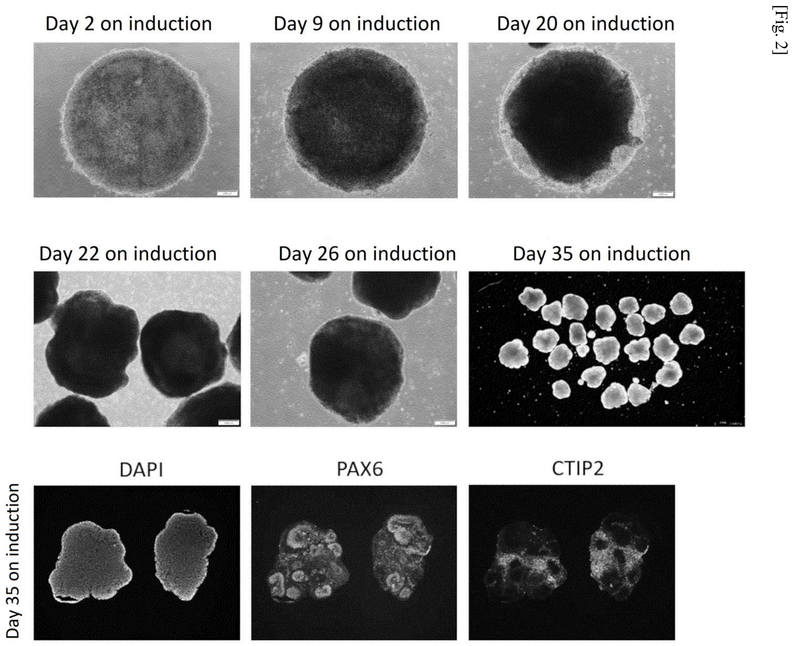

[FIG. 2] FIG. 2 shows the process of the induction of cerebral cortex organoids, and the expression of markers. Human cerebral cortex organoids were produced under conditions where a differentiation-inducing signal SB431542 (5 µM) and a Wnt antagonist IWR-1-endo (3 µM) were removed. When adherent culture of human induced pluripotent stem (iPS) cells (201B7) was conducted on TS-02 (spot diameter: 1,500 µm), the cells on the culturing area could be cultured for 20 days while being kept confluent. The cell morphology was maintained after the cell detachment, and a plurality of layer structures were formed by Day 35 of the culture due to the rotation culture. When the organoids on Day 35 were evaluated through immunostaining, it was confirmed that neural stem cells (positive for PAX6), and cortical plates and fifth/sixth layers of the cerebral cortex (positive for CTIP2) that were formed to surround the neural stem cells were expressed.

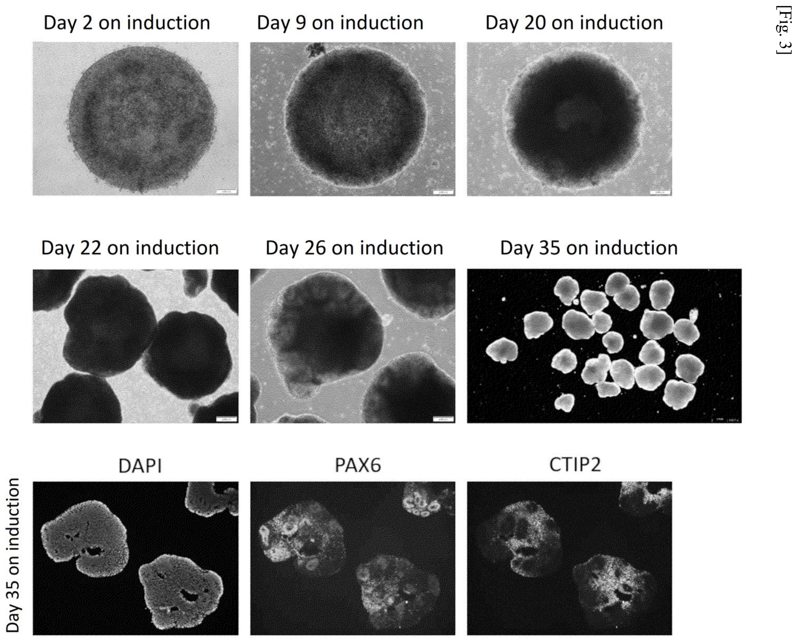

[FIG. 3] FIG. 3 shows the process of the induction of cerebral cortex organoids, and the expression of markers. Human cerebral cortex organoids were produced under conditions where a differentiation-inducing signal SB431542 (5 µM) and a WNT antagonist IWR-1-endo (3 µM) were added. When adherent culture of human iPS cells (201B7) was conducted on TS-02 (spot diameter: 1,500 µm), the cells on the culturing area could be cultured for 20 days while being kept confluent. The cell morphology was maintained after the cell detachment, and a plurality of layer structures were formed by Day 35 of the culture due to the rotation culture. When the organoids on Day 35 were evaluated through immunostaining, it was confirmed that neural stem cells (positive for PAX6), and cortical plates and fifth/sixth layers of the cerebral cortex (positive for CTIP2) that were formed to surround the neural stem cells were expressed.

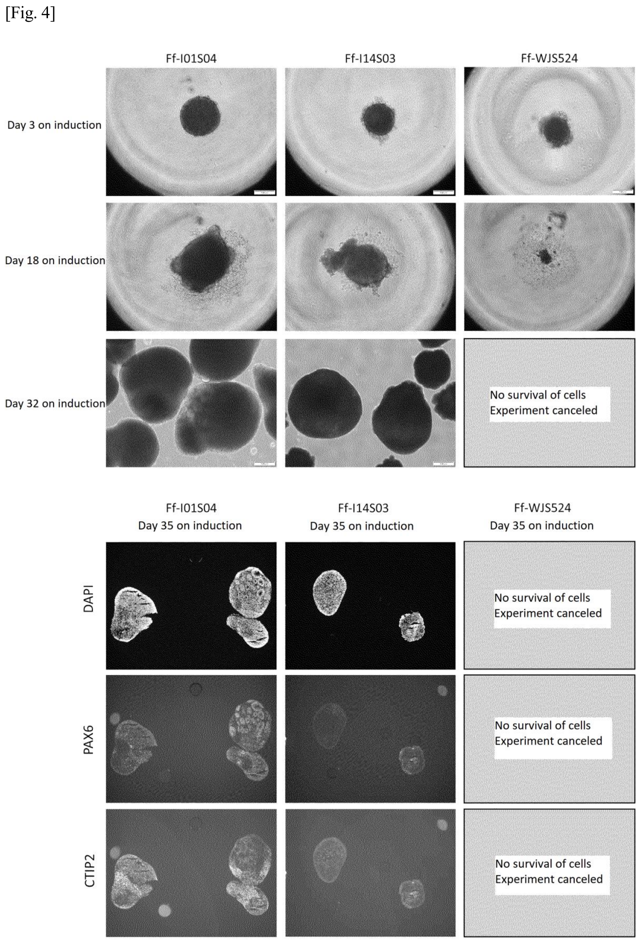

[FIG. 4] FIG. 4 shows the induction of cerebral cortex organoids using a 96-well plate. Human iPS cells (Ff-I01s04 strain, Ff-I14s03 strain, Ff-WJs524 strain) were subjected to induction on a 96-well plate under conditions shown in FIG. 3 where SB431542 (5 µM) and a WNT antagonist IWR-1-endo (3 µM) were added. The difference between the strains was not observed on Day 3 of the differentiation, whereas morphological inequality and proliferation failure (Ff-WJs524 strain) were observed between Days 18 to 32 of the induction. When immunostaining evaluation was made on Day 35, the expression of markers of cortical layers was confirmed in only a portion of the Ff-I01s04 strain, and the expression of the markers could not be confirmed in many cell clusters.

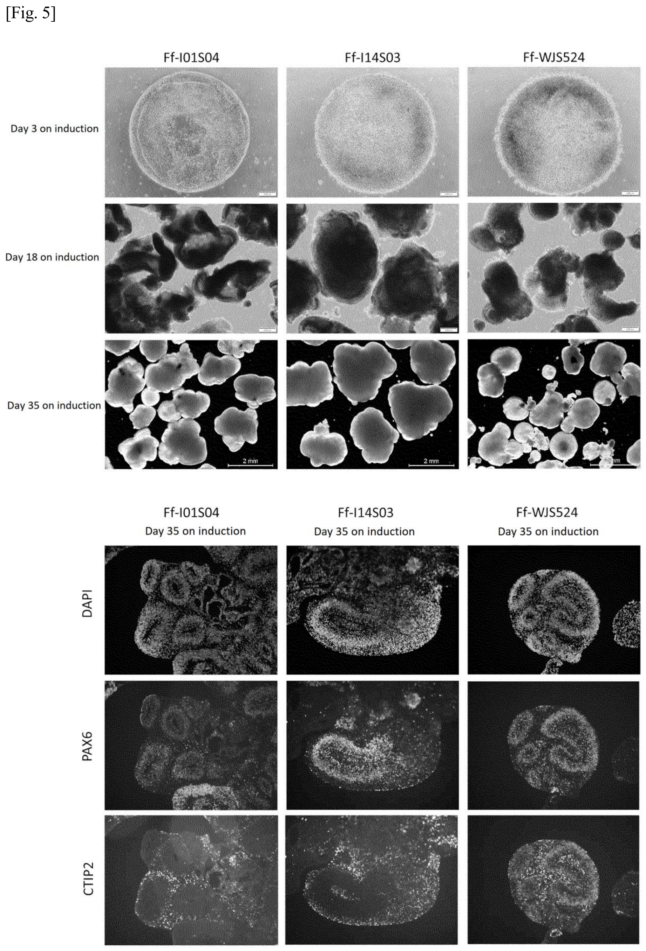

[FIG. 5] FIG. 5 shows the induction of cerebral cortex organoids using a TS-02 plate. Human iPS cells (Ff-I01s04 strain, Ff-I14s03 strain, Ff-WJs524 strain) were subjected to induction on a TS-02 plate under the same conditions as in FIG. 4 above. No cells with proliferation failure or the like were observed before Days 3, 18, and 35 of the differentiation. When immunostaining evaluation was made on Day 35, the expression of markers for neural stem cells (positive for PAX6) and cortical plates (positive for CTIP2) formed to surround the neural stem cells could be confirmed in many cells.

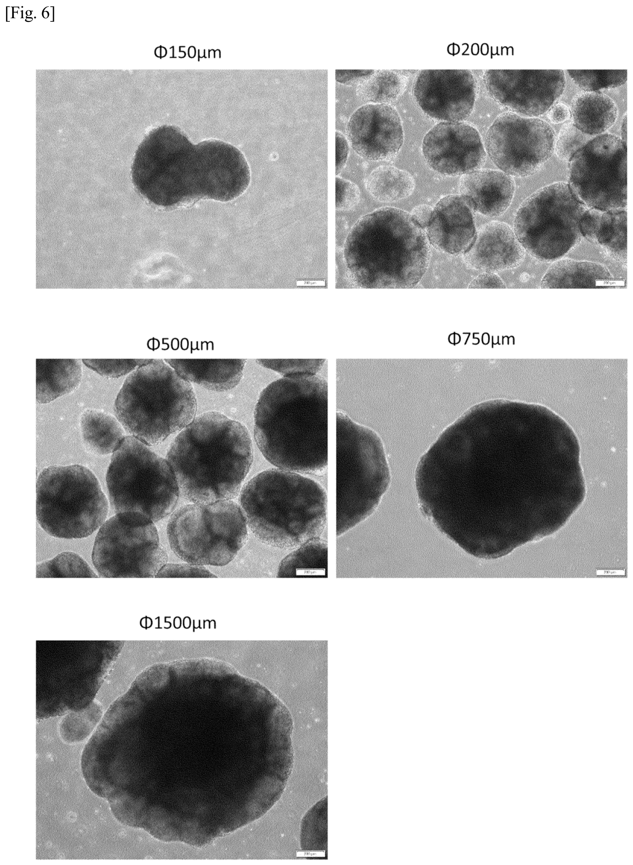

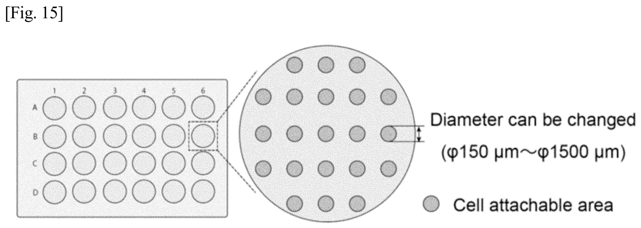

[FIG. 6] FIG. 6 shows evaluations of the induction of cerebral cortex organoids when the micropatterning area of TS-02 was varied. FIG. 15 shows a schematic diagram of TS-02. Human iPS cells (201B7) were subjected to induction under the same conditions as in FIG. 3. Five micropatterning areas with diameters of 150 µm, 200 µm, 500 µm, 750 µm, and 1500 µm on TS-02 were examined. Formation of the layer structure of cerebral cortex organoids was observed on Day 35 of the differentiation induction under all the conditions.

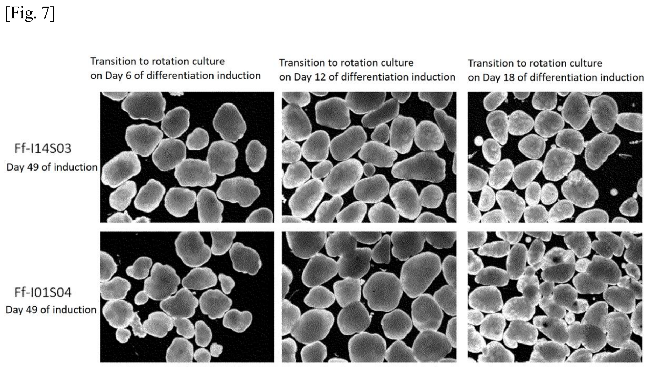

[FIG. 7] FIG. 7 shows an examination made with the timing of the cell detachment from TS-02 being varied. Human iPS cells (Ff-I01s04 strain, Ff-I14s03 strain) were subjected to induction under the same conditions as in FIG. 3. The timing of the cell detachment was Days 6, 12, and 18 of the differentiation induction. When the detached cells were observed on Day 49, the layer structures could be confirmed under all the conditions. Furthermore, it was found that the layer structures were more efficiently formed in the case of transition to rotation culture on Days 12 and 18 of the differentiation induction.

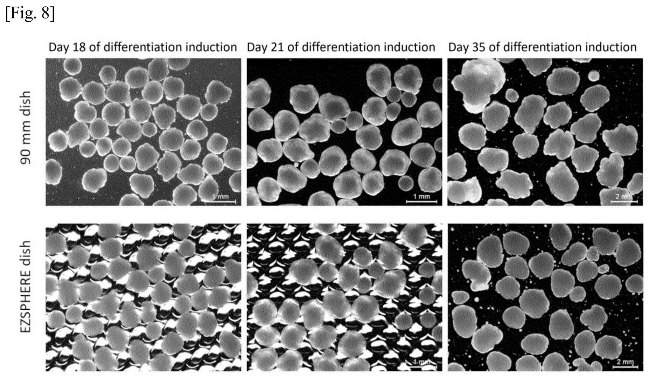

[FIG. 8] FIG. 8 shows equalization of the external appearances of cerebral cortex organoids due to a dish (having projections and depressions). Human iPS cells (201B7) were cultured for 18 days under the same conditions as in FIG. 3. Thereafter, the cells were detached, and were transferred to and cultured on a normal 90-mm dish and a dish (EZSPHERE) having projections and depressions at the timing of rotation culture. When evaluation was made on Day 35 of the induction, it was found that the shapes of the cerebral cortex organoids were more equalized under the conditions where the rotation culture was conducted on EZSPHERE.

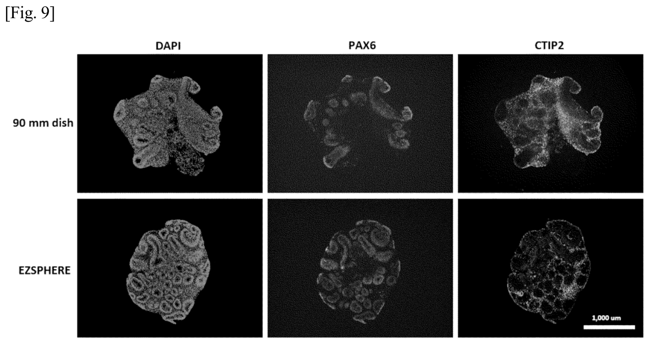

[FIG. 9] FIG. 9 shows equalization of structural distributions of cerebral cortex organoids due to a dish (having projections and depressions). Comparative culture of human ES cells (Kh-ES1) was conducted under the same conditions as in FIG. 8. When immunostaining evaluation was made on Day 35 of the induction, the cerebral cortex layer structures were more equally distributed toward the inside under the conditions where the rotation culture was conducted on EZSPHERE.

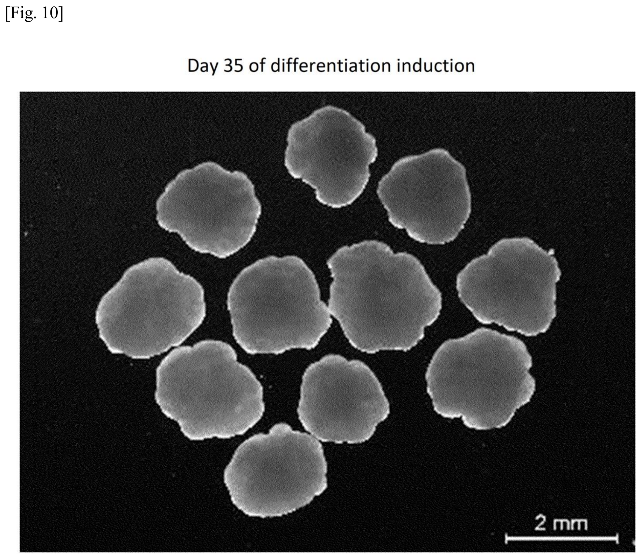

[FIG. 10] FIG. 10 shows a difference in the expression of markers between human cerebral cortex organoids. Human iPS cells (201B7) were cultured under the same conditions as in FIG. 3. The cerebral cortex organoids on Day 35 of the induction were labeled with antibodies against PAX6 and CTIP2, and variations of the expression of the markers in 18 organoids were examined. It was found that the variation of the expression of PAX6 was 27.0±12.7%, and the variation of the expression of CTIP2 was 37.7±10.9%.

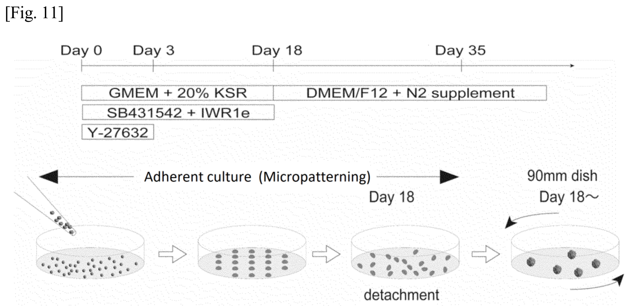

[FIG. 11] FIG. 11 shows the outline of a method for culturing a human cerebral cortex organoid. On Days 0 to 18 of the induction, a cell suspension of iPS cells (B7 strain) (1.2×10⁴ cells/cm²) was seeded, and adherent culture was conducted. The confluent state was maintained inside the cell attachable areas over 18 days. On Day 18 of the induction, the cells were detached and rotation culture was conducted. The cells formed spheres in several days, and cerebral cortex organoids having a neuroepithelial structure were obtained on about Day 35 of the induction.

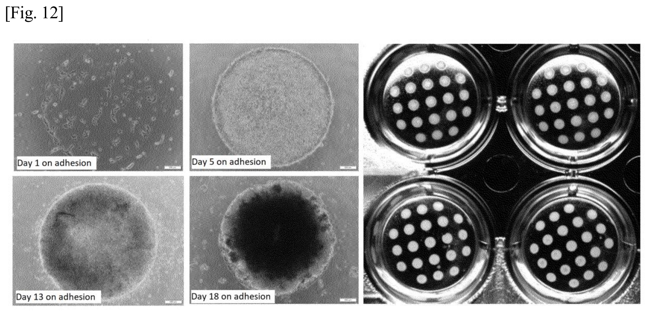

[FIG. 12] FIG. 12 shows the shapes of organoids during the adherent culture process of the culturing method described in FIG. 11. A few cells attached to the attachment surface after the cells had been seeded, and became confluent over about 7 days. Providing 21 attachment surfaces in each well of a 24-well plate made it possible to produce cerebral cortex organoids in large quantities at once.

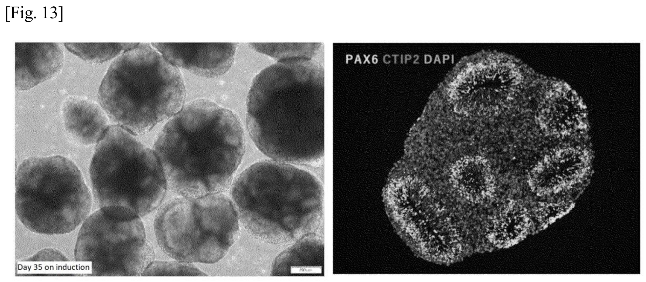

[FIG. 13] FIG. 13 shows the results of the evaluation of structures formed through rotation culture process of the culturing method described in FIG. 11. On Day 18 of the adherent culture, the cells were detached, and rotation culture was conducted thereafter. On Day 17 of the rotation culture (on Day 35 of the total culturing period), structures having a neuroepithelial structure could be confirmed. It was found through immunostaining of the cells thereof that the structures were cerebral cortex organoids expressing a neural stem cell marker (PAX6) and a marker (CTIP2) of the fifth-sixth layer of a cerebral cortex.

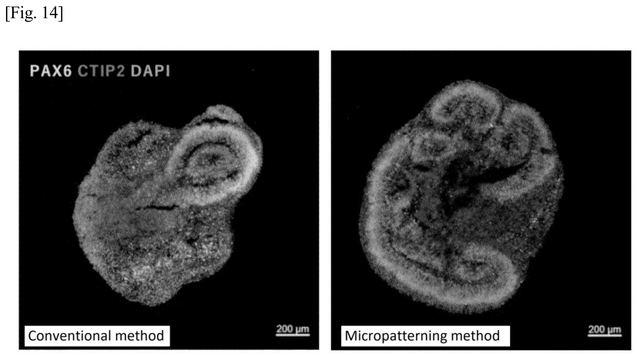

[FIG. 14] FIG. 14 shows the results of comparison between the culture efficiency of a conventional method and the culture efficiency of the culturing method (micropatterning method) described in FIG. 11. Human cerebral cortex organoids were induced using the conventional method and the micropatterning method. When immunostaining evaluation was conducted on Day 35 of the differentiation induction, the structures varied widely between the organoids formed using the conventional method, whereas highly uniform structures were formed using the micropatterning method.
[FIG. 15] FIG. 15 shows a schematic diagram of TS-02.

### DESCRIPTION OF EMBODIMENTS

Hereinafter, an embodiment for carrying out the present invention (referred to merely as "the embodiment" hereinafter) will be described in detail. The embodiment described below is merely an example for describing the present invention, and is not intended to limit the present invention to the contents below. The present invention can be carried out with appropriate modifications within the scope of the present invention.

### 1. Production Method of the Present Invention

The present invention provides a method for producing a neural organoid, the method including:
(1) a step of forming a cell population containing neural stem cells through adherent culture of pluripotent stem cells in a culture vessel that has a plurality of independent cell attachable regions and is configured to have cells that will attach to the regions to be present in the same culture medium; and
(2) a step of detaching the cell population containing neural stem cells formed in the step (1) and conducting suspension culture of the cell population.

### (1) Step (1)

The step (1) of the present invention typically includes:
(1-1) a step of seeding pluripotent stem cells on a culture vessel that has a plurality of independent cell attachable regions and is configured to have cells that will attach to the regions to be present in the same culture medium, and allowing the pluripotent stem cells to attach to the cell attachable regions;
(1-2) a step of conducting expansion culture of the pluripotent stem cells attaching to the cell attachable regions in the step (1-1); and
(1-3) a step of inducing differentiation of the pluripotent stem cells after the expansion culture in the step (1-2) into a cell population containing neural stem cells.

### (2) Culture Vessel and Cells

In this specification, the term "cell attachable regions" refers to regions to which pluripotent stem cells have a higher adhesion compared with a region other than these regions. In this specification, the region other than the cell attachable regions in a culture vessel may be referred to as a "cell non-attachable region". Also, in the present invention, the term "independent cell attachable regions" refers to a state in which the cell attachable regions in the culture vessel are not in contact with one another. In this specification, the term "cell attachable regions" and the term "micropatterning areas" are used interchangeably.

In this specification, the wording "have cells that will attach to the regions to be present in the same culture medium" is not particularly limited as long as the cells share a culture medium in the culture vessel. Without being bound by any theory, it is presumed that by conducting culture, as in the present invention, with a culture medium shared, cells (or cell populations) present on the cell attachable regions in the culture vessel become uniform (equal) due to the paracrine effect, resulting in stable (equal) quality of neural organoids.

In this specification, the term "stem cell" means an undifferentiated cell having a differentiation potential and a proliferative capacity (particularly a self-proliferative capacity). The stem cell includes a pluripotent stem cell, a multipotent stem cell, a unipotent stem cell, and the like classified based on the differentiation potential. The term "pluripotent stem cell" refers to a stem cell that can be cultured in vitro and has the potential (pluripotency) of differentiating into all types of cells included in a living organism. The wording "all types of cells" means cells derived from three germ layers, namely the ectoderm, the mesoderm, and the endoderm. The term "multipotent stem cell" means a stem cell having the potential of differentiating into not all types but a plurality of types of tissues or cells. The term "unipotent stem cell" means a stem cell having the potential of differentiating into a specific type of tissue or cell.

The pluripotent stem cell can be induced from a fertilized egg, a cloned embryo, a germline stem cell, a tissue stem cell, somatic cell, and the like. Examples of the pluripotent stem cell include an embryonic stem cell (ES cell), an embryonic germ cell (EG cell), an induced pluripotent stem cell (iPS cell), and the like. The pluripotent stem cell also encompasses a multi-lineage differentiating stress enduring cell (Muse cell) obtained from a mesenchymal stem cell (MSC), and a GS cell produced from a germ cell (e.g., testis). When the pluripotent stem cell is an embryonic stem cell or an arbitrary cell derived from a human embryo, the cell may be obtained by destruction of the embryo or they may be obtained without destruction of the embryo, but preferably they are cells obtained without destruction of the embryo from an ethical viewpoint. Also, a human embryonic stem cell may be established from a human embryo within 14 days after fertilization.

The embryonic stem cell was established for the first time in 1981, and has been applied in the production of knock-out mice since 1989. In 1998, the human embryonic stem cell was established and are also being utilized in regenerative medicine. The ES cells can be produced by culturing an inner cell population on feeder cells or in a culture medium containing a leukemia inhibitory factor (LIF). The methods for producing ES cells are described in, for example, WO 96/22362, WO 02/101057, US Patent No. 5843780, US Patent No. 6200806, US Patent No. 6280718, and the like. The embryonic stem cells can be obtained from a prescribed institute, and commercially available products thereof can also be purchased. For example, human embryonic stem cells KhES-1, KhES-2, and KhES-3 are available from the Institute for Frontier Medical Sciences of Kyoto University. The EB5 cells and the D3 strain, which are both mouse embryonic stem cells, are available from the Institute of Physical and Chemical Research and the American Type Culture Collection (ATCC), respectively. A nuclear-transfer ES cell (ntES cell), which is one type of ES cell, can be established from a cloned embryo produced by transplanting a cell nucleus of a somatic cell into an egg cell from which a cell nucleus has been removed.

The EG cells can be produced by culturing primordial germ cells in a culture medium containing a mouse stem-cell factor (mSCF), an LIF, and a basic fibroblast growth factor (bFGF) (Cell, 70: 841-847, 1992).

The "induced pluripotent stem cell" is a cell with pluripotency induced through reprogramming of a somatic cell using a known method or the like. Specific examples of the induced pluripotent stem cell include cell with multipotency induced through reprogramming of somatic cells that have differentiated into fibroblasts, peripheral blood monocytes, and the like caused by expression of a plurality of genes selected from an initialization gene group that includes Oct3/4, Sox2, Klf4, Myc (c-Myc, N-Myc, L-Myc), Glis1, Nanog, Sall4, lin28, Esrrb, etc. In 2006, Yamanaka, et al. established induced pluripotent stem cells using mouse cells (Cell, 2006, 126(4) pp.663-676). The induced pluripotent stem cells were also established using human fibroblasts in 2007, and these induced pluripotent stem cells have pluripotency and a self-proliferative capacity similarly to embryonic stem cells (Cell, 2007, 131(5) pp.861-872; Science, 2007, 318(5858) pp.1917-1920; Nat. Biotechnol., 2008, 26(1) pp.101-106). In addition to a method for producing induced pluripotent stem cells through direct reprogramming caused by gene expression, induced pluripotent stem cells can also be induced from somatic cells through addition of compounds, or the like (Science, 2013, 341, pp.651-654).

Somatic cells used to produce induced pluripotent stem cells are not particularly limited, and examples thereof include tissue-derived fibroblasts, hemocyte cells (e.g., peripheral blood monocytes, T cells, and the like), hepatocytes, pancreatic cells, intestinal epithelium cells, smooth muscle cells, and the like.

When reprogramming is caused by expression of some types of genes (e.g., four factors of Oct3/4, Sox2, Klf4, and Myc) in order to produce induced pluripotent stem cells, a means for expressing the genes is not particularly limited. Examples of the means for expressing the genes include infection techniques in which a viral vector (e.g., a retroviral vector, a lentiviral vector, a Sendai viral vector, an adenoviral vector, or an adeno-associated viral vector) is used, gene transfer techniques (e.g., a calcium phosphate technique, a lipofection technique, a RetroNectin technique, and an electroporation technique) in which a plasmid vector (e.g., a plasmid vector or an episomal vector) is used, gene transfer techniques (e.g., a calcium phosphate technique, a lipofection technique, and an electroporation technique) in which an RNA vector is used, direct protein injection techniques, and the like.

Established induced pluripotent stem cells are also available, and, for example, human induced pluripotent cell strains such as 201B7 cells, 201B7-Ff cells, 253G1 cells, 253G4 cells, 1201C1 cells, 1205D1 cells, 1210B2 cells, and 1231A3 cells, which were established in Kyoto University, are available from Kyoto University and iPS Academia Japan, Inc. For example, Ff-I01 cells, Ff-I01s04 cells, Ff-I14 cells, Ff-I14s03 cells, QHJI01s04 cells, Ff-WJs524 cells, and the like, which were established in Kyoto University, are available from Kyoto University as the established induced pluripotent stem cells.

The pluripotent stem cells may be genetically modified. Genetically modified pluripotent stem cells can be produced using, for example, a homologous recombination technique. Examples of a gene on the chromosome to be modified include cell marker genes, histocompatibility antigen genes, genes related to diseases (e.g., neurodegenerative diseases) caused by neural cell damage, and the like. A target gene on the chromosome can be modified using methods described in "Manipulating the Mouse Embryo, A Laboratory Manual, Second Edition", Cold Spring Harbor Laboratory Press (1994), "Gene Targeting, A Practical Approach", IRL Press at Oxford University Press (1993), "Bio-Manual Series 8, Gene Targeting, Production of Mutant Mice Using ES Cells", Yodosha Co., Ltd. (1995), and the like.

Specifically, for example, the genomic gene of a modification target gene (e.g., a cell marker gene, a histocompatibility antigen gene, or a gene related to a disease (e.g., neurodegenerative disease)) is isolated, and a target vector for conducting homologous recombination on the target gene is produced using the isolated genomic gene. Stem cells with a modified gene on the chromosome can be produced by introducing the produced target vector into stem cells and selecting cells in which homologous recombination has occurred between the target gene and the target vector.

Examples of the method for isolating the genomic gene of a target gene include known methods described in "Molecular Cloning, A Laboratory Manual, Second Edition", Cold Spring Harbor Laboratory Press (1989), "Current Protocols in Molecular Biology", John Wiley & Sons (1987-1997), and the like. The genomic DNA library screening system (manufactured by Genome Systems Inc.), Universal GenomeWalker Kits (manufactured by Clontech Laboratories, Inc.), and the like can be used.

The production of the target vector for conducting homologous recombination on the target gene and the effective selection of the homologous recombinant can be conducted in accordance with the methods described in "Gene Targeting, A Practical Approach", IRL Press at Oxford University Press (1993), "Bio-Manual Series 8, Gene Targeting, Production of Mutant Mice Using ES Cells", Yodosha Co., Ltd. (1995), and the like. The target vector may be of a replacement type or an insertion type. The selection method may be a method such as positive selection, promoter selection, negative selection, or poly-A selection. The method for selecting a homologous recombinant of interest from selected cell strains may be, for example, southern hybridization with genomic DNA, the PCR technique, or the like.

Genome-edited pluripotent stem cells can also be used as the pluripotent stem cells. The "genome editing" is a technique for intentionally modifying a target gene or a genomic region by utilizing principles such as site-specific cleavage of a genomic DNA strand with a nuclease, and chemical base conversion. Examples of the site-specific nuclease include a zinc finger nuclease (ZFN), TALEN, a CRISPR/Cas9 system, and the like. The genome editing technique can be used to produce a knock-out cell strain obtained by deleting a specific gene, a knock-in cell strain obtained by artificially inserting another sequence into a specific gene locus, and the like.

The pluripotent stem cells may be disease-specific pluripotent stem cells. The term "disease-specific pluripotent stem cells" refers to pluripotent stem cells with gene modification or a genetic background related to development of a disease. The disease-specific pluripotent stem cells can be produced using a method for establishing induced pluripotent stem cells from a patient suffering from a target diseases or his/her relatives through the method described above or the like, a method for modifying the genome of a previously established pluripotent stem cell through a genome editing technique or the like such as a zinc finger nuclease (ZFN), TALEN, or a CRISPR/CAS system, or the like. Examples of the disease include a neurodegenerative disease and the like.

The pluripotent stem cells used in the present invention are, for example, pluripotent stem cells of a homoiothermic animal and preferably pluripotent stem cells of a mammal, and examples of the mammal include rodents, ungulates, those belonging to the order Carnivora, those belonging to the order Lagomorpha, primates, and the like. Examples of the rodents include a mouse, a rat, a hamster, a guinea pig, and the like. Examples of the ungulates include a pig, a cow, a goat, a horse, a sheep, and the like. Examples of the mammals belonging to the order Carnivora include a dog, a cat, and the like. Examples of the mammals belonging to the order Lagomorpha include a rabbit and the like. The term "primates" refer to mammals belonging to the order Primates, and examples of the primates include mammals belonging to the suborder Prosimii such as a lemur, a loris, and a tree shrew, and mammals belonging to the suborder Anthropoidea such as a monkey, an anthropoid, and a human. The pluripotent stem cells are preferably pluripotent stem cells of a rodent (e.g., a mouse or a rat) or a primate (e.g., a human or a monkey), and more preferably human pluripotent stem cells.

The term "adherent culture" refers to culturing cells while maintaining a state in which the cells attach to a culture vessel or the like. The attachment may be attachment through hydrophobic interaction, electrostatic interaction, or physicochemical interaction caused by van der Waals force or the like, or attachment via a cell attachment protein of an extracellular matrix and the like.

In the present invention, a culture vessel used for adherent culture has a plurality of independent cell attachable regions and is configured to have cells that will attach to the regions to be present in the same culture medium. There is no particular limitation on the culture vessel as long as the configuration above can be satisfied, and examples thereof include a flask, a tissue culture flask, a dish, a tissue culture dish, a multi-dish, a microplate, a microwell plate, a micropore, a multi-plate, a multi-well plate, a chamber slide, a laboratory dish, a tube, a tray, a culture bag, a microcarrier, a bead, a stacking plate, a spinner flask, a roller bottle, and the like.

The area and number of the cell attachable regions of the culture vessel of the present invention used for the adherent culture are not particularly limited as long as neural organoids with desired quality are obtained through suspension culture in the step (2) of the present invention, but the area thereof is, for example, 0.005 to 10 mm², preferably 0.01 to 5.0 mm², and more preferably 0.04 to 2.25 mm². The number of the cell attachable regions in the culture vessel is, for example, 1 or more, and preferably 5 or more (10, 50, 100, 200, 300, 400, 500, 1000, or more). The shape of the regions is not particularly limited, but is may be, for example, a circular shape, an elliptical shape, a rectangular shape, a regular polygonal shape, or the like from the viewpoint of improving the differentiation inducing efficiency. In an aspect, the cell attachable regions having a circular shape preferably have a diameter of 150 µm to 1500 µm.

The surfaces of the cell attachable regions of the culture vessel used in the present invention may be artificially treated for the purpose of improving the adhesion to the cells (promoting the adhesion to the cells). Also, the surface of a region (cell non-attachable region other than the cell attachable regions of the culture vessel may be artificially treated for the purpose of reducing the adhesion to the cells (inhibiting the adhesion to the cells). Examples of the artificial treatment include coating with an extracellular matrix, a polymer (e.g., a hydrophilic polymer, a hydrophobic polymer, or a temperature-responsive polymer), or the like, and surface processing such as gas plasma treatment or positive charge treatment. Examples of the extracellular matrix to which the cells are attachable include a basement membrane preparation, laminin, entactin, collagen, gelatin, and the like. Examples of the polymer include polylysine, polyornithine, and the like. More specifically, treatment as follows may be conducted.

In an aspect, the culture vessel used in the present invention has a configuration in which a hydrophilic polymer layer is contained on the surface, the cell attachable regions are regions in which a portion of the hydrophilic polymer layer is decomposed or reformed through any of plasma treatment, ultraviolet treatment, and corona discharge treatment, or a combination thereof, and adsorption of a protein that contributes to the culture vessel-cell attachment on this region can be suppressed due to the cell non-attachable region has the hydrophilic polymer layer on the surface.

The thickness of the hydrophilic polymer layer is not particularly limited as long as the cell attachable regions and the cell non-attachable region are formed through the treatment above, but the thickness thereof is, for example, 10 nm or more, preferably 50 nm or more, more preferably 100 nm or more, and even more preferably 500 nm or more. Also, the thickness of the cell attachable regions of the culture vessel is not particularly limited as long as the adhesion of pluripotent stem cells thereto is higher compared with the cell non-attachable region, but the thickness thereof is, for example, 1000 nm or less, preferably 500 nm or less, more preferably 100 nm or less, and even more preferably 50 nm or less.

The hydrophilic polymer layer can be formed using at least one of a method of forming a chemical bond and a method in which physical interaction is utilized. Examples of the method of forming a chemical bond include techniques for forming a reactive functional group, such as ultraviolet irradiation, electron beam irradiation, gamma ray irradiation, plasma treatment, and corona treatment. It is also possible to conduct crosslinking reaction for the substrate surface by utilizing organic reaction with ions or radicals used as a reaction source. As the method in which physical interaction is utilized, it is possible to use techniques in which a matrix with excellent compatibility with a target hydrophilic polymer is used as a coating material, such as application, brush application, dip coating, spin coating, bar coating, flow coating, spray coating, roll coating, air knife coating, blade coating, gravure coating, microgravure coating, and slot die coating.

The type of the hydrophilic polymer is not particularly limited, but examples thereof include those having a polar group such as a hydroxy group, an amino group, or a polyethylene glycol group, and those having a zwitterionic structure such as a betaine structure or a phosphorylcholine group. In order to form the cell non-attachable region, for example, a hydroxy group, a phosphorylcholine group, or a polyethylene glycol group is preferable, a hydroxy group or a phosphorylcholine group is more preferable, and a phosphorylcholine group is even more preferable.

The hydrophilic polymer is preferably a random or block copolymer having both a hydrophilic monomer unit and a hydrophobic monomer unit, and more preferably a random copolymer having both a hydrophilic monomer unit and a hydrophobic monomer unit, from the viewpoint of, for example, suppressing leaching of the hydrophilic polymer from the culture vessel and reduce impact of mixing of the polymer into a cell aggregate and the like on the quality. Also, the composition ratio of the hydrophilic monomer unit in the copolymer, for example, preferably 30 wt% or more, more preferably 40 wt% or more, even more preferably 50 wt% or more, and even more preferably 60 wt% or more, in order to form a favorable cell non-attachable region. The composition ratio of the hydrophobic monomer unit is, for example, preferably 20 wt% or more, more preferably 30 wt% or more, even more preferably 40 wt% or more, and even more preferably 50 wt% or more, from the viewpoint of suppressing the leaching of the hydrophilic polymer.

The hydrophilic monomer unit is not particularly limited, except that it is hydrophilic, and examples thereof include those having an amino group, such as 2-dimethylaminoethyl acrylate, 2-dimethylaminoethyl methacrylate, 2-diethylaminoethyl acrylate, 2-diethylaminoethyl methacrylate, and N-[3-(dimethylamino)propyl] acrylamide; those having betaine, such as N-(3-sulfopropyl)-N-methacryloyloxyethyl-N,N-dimethylammonium betaine and N-methacryloyloxyethyl-N,N-dimethylammonium-α-N-methyl carboxybetaine; those having a polyethylene glycol group and a methoxyethyl group, such as hydroxyethyl acrylate, hydroxyethyl methacrylate, N-(2-hydroxyethyl) acrylamide, polyethylene glycol monoacrylate, polyethylene glycol monomethacrylate, polypropylene glycol monoacrylate, polypropylene glycol monomethacrylate, methoxypolyethylene glycol monoacrylate, methoxypolyethylene glycol monomethacrylate, diethylene glycol monomethyl ether acrylate, diethylene glycol monomethyl ether methacrylate, diethylene glycol monoethyl ether acrylate, diethylene glycol monoethyl ether methacrylate, 2-methoxyethyl acrylate, 2-methoxyethyl methacrylate, 2-ethoxyethyl acrylate, 2-ethoxyethyl methacrylate, 3-butoxyethyl acrylate, 3-butoxyethyl methacrylate, 3-butoxyethyl acrylamide, furfuryl acrylate, furfuryl methacrylate, tetrahydrofurfuryl acrylate, and tetrahydrofurfuryl methacrylate; those having an acrylate group, such as methoxymethyl acrylate, methoxymethyl methacrylate, 2-ethoxymethyl acrylate, 2-ethoxymethyl methacrylate, 3-butoxymethyl acrylate, 3-butoxymethyl methacrylate, and 3-butoxymethyl acrylamide; and those having a phosphorylcholine group, such as 2-methacryloyloxyethyl phosphorylcholine, 2-acryloyloxyethyl phosphorylcholine, 3-(meth)acryloyloxypropyl phosphorylcholine, 4-(meth)acryloyloxybutyl phosphorylcholine, 6-(meth)acryloyloxyhexyl phosphorylcholine, 10-(meth)acryloyloxydecyl phosphorylcholine, ω-(meth)acryloyl(poly)oxyethylene phosphorylcholine, 2-acrylamidoethyl phosphorylcholine, 3-acrylamidopropyl phosphorylcholine, 4-acrylamidobutyl phosphorylcholine, 6-acrylamidohexyl phosphorylcholine, 10-acrylamidodecyl phosphorylcholine, and ω-(meth)acrylamide(poly)oxyethylene phosphorylcholine.

The hydrophobic monomer unit is not particularly limited, except that it is hydrophobic, and examples thereof include n-butyl acrylate, n-butyl methacrylate, isobutyl acrylate, isobutyl methacrylate, t-butyl acrylate, t-butyl methacrylate, n-hexyl acrylate, n-hexyl methacrylate, n-octyl acrylate, n-octyl methacrylate, n-decyl acrylate, n-decyl methacrylate, n-dodecyl acrylate, n-dodecyl methacrylate, n-tetradecyl acrylate, n-tetradecyl methacrylate, and the like.

It is preferable that the hydrophilic polymer contains, for example, a reactive monomer unit from the viewpoint of suppressing leaching of the hydrophilic polymer. The reactive monomer unit is preferably a monomer unit with UV reactivity because the hydrophilic polymer can be immobilized on the culture vessel through brief treatment, and examples thereof include 4-azidophenyl acrylate, 4-azidophenyl methacrylate, 2-((4-azidobenzoyl)oxy)ethyl acrylate, 2-((4-azidobenzoyl)oxy)ethyl methacrylate, and the like.

In an aspect of the culture vessel used in the present invention, the cell attachable regions may have temperature responsiveness for the purpose of reducing damage caused by detachment of cultured neural organoids. When the cell attachable regions have temperature responsiveness, the response temperature is preferably 50°C or lower and more preferably 35°C or lower because cells can be cultured at a temperature close to the body temperature during the culture of the cells on the culture vessel. Also, the response temperature is particularly preferably 25°C or lower because it is suitable for suppressing detachment of cells when conducting an operation such as replacement of the culture medium or the like during the culture. Furthermore, the response temperature is preferably 4°C or higher, more preferably 10°C or higher, and even more preferably 15°C or higher because neural organoids can be formed through a cooling operation conducted at a temperature that does not cause damage on the cells. The temperature responsiveness may be achieved, for example, by using a temperature-responsive polymer, which will be described below.

In this specification, the term "temperature-responsive polymer" refers to a polymer that changes in the degree of hydrophilicity/hydrophobicity in response to a change in temperature.

Also, when the cell attachable regions have temperature responsiveness, they may further include a layer with a thickness of 1 to 100 nm containing a temperature-responsive polymer, for example, on the surface of the layer containing the hydrophilic polymer. Due to the layer with a thickness of 1 to 100 nm containing a temperature-responsive polymer being included, the cell attachable regions can be provided with the temperature responsiveness without impairing the characteristics of the cell attachable regions and the cell non-attachable region formed on the surface of the layer containing the hydrophilic polymer. The thickness of the layer containing the temperature-responsive polymer is more preferably 3 to 50 nm, even more preferably 5 to 40 nm, and most preferably 10 to 35 nm, because it is suitable for providing the temperature responsiveness to the cell attachable regions without impairing the characteristics of the cell attachable regions and the cell non-attachable region.

A block copolymer described in Examples, which will be described below, has a water-insoluble block segment and thus is insoluble in water, but is a temperature-responsive polymer with a response temperature in which the degree of hydrophilicity/hydrophobicity changes in response to a change in temperature. To determine the response temperature of the block copolymer, a substrate coated with the block copolymer is immersed in water, and the degree of hydrophilicity/hydrophobicity of the block copolymer is measured by measuring the contact angle of a bubble in the water. Next, the temperature of the block copolymer is changed by changing the temperature of the water, and the block copolymer is left to stand until the temperature becomes stable. Then, the contact angle of a bubble is measured again, and thus the contact angles at various temperatures are determined. A curve as follows is obtained: the contact angle of a bubble is a large value (the contact angle with water is a small value) and is substantially constant at temperatures lower than and equal to the response temperature, but starts to decrease (the contact angle with water becomes a larger value) at the response temperature, and is substantially constant at temperatures higher than and equal to the response temperature. The response temperature can be determined by determining a temperature at which the contact angle is an average value of the contact angle at temperatures lower than or equal to the response temperature and the contact angle at temperatures higher than and equal to the response temperature (midpoint method). The temperature range for the measurement includes a temperature width of 10°C or more that is lower than or equal to the response temperature and in which the contact angle is substantially constant as well as a temperature width of 10°C or more that is higher than or equal to the response temperature and in which the contact angle is substantially constant.

The temperature-responsive polymer is preferably a block copolymer having a water-insoluble block segment and a temperature-responsive block segment. Using such a block copolymer as the temperature-responsive polymer makes it possible to improve the mass productivity of the culture vessel and to suppress mixing of the temperature-responsive polymer into the neural organoids produced. The constitutional unit ratio of the temperature-responsive block segment contained in the temperature-responsive polymer is preferably 70 wt% or more, more preferably 80 wt% or more, particularly preferably 90 wt% or more, and most preferably 92 wt% or more because it is suitable for rapidly detaching the neural organoids from the culture vessel.

Examples of the monomer unit included in the temperature-responsive block segment include (meth)acrylamide compounds such as acrylamide and methacrylamide; N-alkylsubstituted (meth)acrylamide derivatives such as N,N-diethyl acrylamide, N-ethyl acrylamide, N-n-propyl acrylamide, N-n-propyl methacrylamide, N-isopropyl acrylamide, N-isopropyl methacrylamide, N-cyclopropyl acrylamide, N-cyclopropyl methacrylamide, N-t-butyl acrylamide, N-ethoxyethyl acrylamide, N-ethoxyethyl methacrylamide, N-tetrahydrofurfuryl acrylamide, and N-tetrahydrofurfuryl methacrylamide; N,N-dialkyl-substituted (meth)acrylamide derivatives such as N,N-dimethyl (meth)acrylamide, N,N-ethylmethyl acrylamide, and N,N-diethyl acrylamide; (meth)acrylamide derivatives having a cyclic group such as 1-(1-oxo-2-propenyl)-pyrrolidine, 1-(1-oxo-2-propenyl)-piperidine, 4-(1-oxo-2-propenyl)-morpholine, 1-(1-oxo-2-methyl-2-propenyl)-pyrrolidine, 1-(1-oxo-2-methyl-2-propenyl)-piperidine, and 4-(1-oxo-2-methyl-2-propenyl)-morpholine; vinyl ethers such as methyl vinyl ether; and proline derivatives such as N-proline methyl ester acrylamide. N,N-diethyl acrylamide, N-n-propyl acrylamide, N-isopropyl acrylamide, N-n-propyl methacrylamide, N-ethoxyethyl acrylamide, N-tetrahydrofurfuryl acrylamide, and N-tetrahydrofurfuryl methacrylamide are preferable, N-n-propyl acrylamide, and N-isopropyl acrylamide are more preferably, and N-isopropyl acrylamide is particularly preferable, because these are suitable for setting the response temperature to 0°C to 50°C. When a culture medium at room temperature is used in replacement of the culture medium during the culturing operation, N-n-propyl acrylamide and N-proline methyl ester acrylamide are preferable because these are suitable for setting the response temperature of the block copolymer to a temperature lower than room temperature.

Examples of the monomer unit included in the water-insoluble block segment include n-butyl acrylate, n-butyl methacrylate, isobutyl acrylate, isobutyl methacrylate, t-butyl acrylate, t-butyl methacrylate, n-hexyl acrylate, n-hexyl methacrylate, n-octyl acrylate, n-octyl methacrylate, n-decyl acrylate, n-decyl methacrylate, n-dodecyl acrylate, n-dodecyl methacrylate, n-tetradecyl acrylate, n-tetradecyl methacrylate, and the like. A monomer unit having a reactive group is preferable because it is suitable for firmly immobilizing the block copolymer to the substrate, and examples thereof include 4-azidophenyl acrylate, 4-azidophenyl methacrylate, 2-((4-azidobenzoyl)oxy)ethyl acrylate, 2-((4-azidobenzoyl)oxy)ethyl methacrylate, and the like. Furthermore, a structure having an aromatic ring is preferable because it is suitable for improving the cell proliferative effects, and examples thereof include 2-hydroxyphenyl acrylate, 2-hydroxyphenyl methacrylate, 3-hydroxyphenyl acrylate, 3-hydroxyphenyl methacrylate, 4-hydroxyphenyl acrylate, 4-hydroxyphenyl methacrylate, N-(2-hydroxyphenyl)acrylamide, N-(2-hydroxyphenyl)methacrylamide, N-(3-hydroxyphenyl)acrylamide, N-(3-hydroxyphenyl)methacrylamide, N-(4-hydroxyphenyl)acrylamide, N-(4-hydroxyphenyl)methacrylamide, styrene, and the like.

The water-insoluble block segment may also contain a repeating unit for controlling the response temperature of the block copolymer. The repeating unit for controlling the response temperature of the block copolymer is not particularly limited and may be a hydrophilic or hydrophobic component. Examples thereof include, as described above, those having an amino group, such as 2-dimethylaminoethyl acrylate, 2-dimethylaminoethyl methacrylate, 2-diethylaminoethyl acrylate, 2-diethylaminoethyl methacrylate, and N-[3-(dimethylamino)propyl]acrylamide; those having betaine, such as N-(3-sulfopropyl)-N-methacryloyloxyethyl-N,N-dimethylammonium betaine and N-methacryloyloxyethyl-N,N-dimethylammonium-α-N-methyl carboxybetaine; those having a polyethylene glycol group and a methoxyethyl group, such as hydroxyethyl acrylate, hydroxyethyl methacrylate, N-(2-hydroxyethyl)acrylamide, polyethylene glycol monoacrylate, polyethylene glycol monomethacrylate, polypropylene glycol monoacrylate, polypropylene glycol monomethacrylate, methoxypolyethylene glycol monoacrylate, methoxypolyethylene glycol monomethacrylate, diethylene glycol monomethyl ether acrylate, diethylene glycol monomethyl ether methacrylate, diethylene glycol monoethyl ether acrylate, diethylene glycol monoethyl ether methacrylate, 2-methoxyethyl acrylate, 2-methoxyethyl methacrylate, 2-ethoxyethyl acrylate, 2-ethoxyethyl methacrylate, 3-butoxyethyl acrylate, 3-butoxyethyl methacrylate, 3-butoxyethyl acrylamide, furfuryl acrylate, furfuryl methacrylate, tetrahydrofurfuryl acrylate, and tetrahydrofurfuryl methacrylate; those having an acrylate group, such as methoxymethyl acrylate, methoxymethyl methacrylate, 2-ethoxymethyl acrylate, 2-ethoxymethyl methacrylate, 3-butoxymethyl acrylate, 3-butoxymethyl methacrylate, and 3-butoxymethyl acrylamide; and those having a phosphorylcholine group, such as 2-methacryloyloxyethyl phosphorylcholine, 2-acryloyloxyethyl phosphorylcholine, 3-(meth)acryloyloxypropyl phosphorylcholine, 4-(meth)acryloyloxybutyl phosphorylcholine, 6-(meth)acryloyloxyhexyl phosphorylcholine, 10-(meth)acryloyloxydecyl phosphorylcholine, ω-(meth)acryloyl(poly)oxyethylene phosphorylcholine, 2-acrylamidoethyl phosphorylcholine, 3-acrylamidopropyl phosphorylcholine, 4-acrylamidobutyl phosphorylcholine, 6-acrylamidohexyl phosphorylcholine, 10-acrylamidodecyl phosphorylcholine, and ω-(meth)acrylamide(poly)oxyethylene phosphorylcholine.

Other examples of the method for producing the culture vessel used for the adherent culture of the present invention include a method in which a partial region of the culture vessel is coated with a substance that promotes or inhibits cell adhesion through photolithography or an inkjet technique, a method in which the surface of the culture vessel is treated through any of plasma treatment, ultraviolet treatment, and corona discharge treatment, or a combination thereof and is then coated with the temperature-responsive polymer, and the like.

The culture vessel used for the adherent culture of the present invention may also be sterilized. The sterilization method is not particularly limited, but examples thereof include high-pressure steam sterilization, UV sterilization, γ-ray sterilization, ethylene oxide gas sterilization, and the like. For example, high-pressure steam sterilization, UV sterilization, or ethylene oxide gas sterilization is preferable from the viewpoint of suppressing degeneration of the block copolymer, UV sterilization, or ethylene oxide gas sterilization is more preferable from the viewpoint of suppressing deformation of the substrate, and ethylene oxide gas sterilization is even more preferable because excellent mass productivity of the culture vessel is achieved.

In the present invention, the material used to produce the culture vessel is not particularly limited, but examples thereof include commonly used glass, polymer compounds such as polystyrene, polycarbonate, polyethylene terephthalate, polyvinylidene fluoride, polyethylene, polypropylene, and polyethylenemethacrylate, ceramics, and metals. Polystyrene is preferable because it has excellent transparency, and molding and surface reforming thereof can be easily conducted.

As described above, the cell attachable regions of the culture vessel used for the adherent culture of the present invention may be coated with extracellular matrix such as a basement membrane preparation, fibronectin, laminin or a fragment thereof, entactin, collagen, gelatin, Synthemax, vitronectin, and the like. The exemplified extracellular matrix may be natural one, or that artificially synthesized using a gene recombination technology or the like, or a fragment formed through cleavage by a restriction enzyme or the like, or a synthesized protein or synthesized peptide based on these substances derived from a living organism. Also, as described below, the culture vessel used in the step (1) may be coated with the extracellular matrix in advance, or the culture vessel may be coated with the extracellular matrix by adding the extracellular matrix in the culture medium used in the step (1).

The term "basement membrane structure" means a thin membrane-like structure composed of the extracellular matrix. In vivo, the basement membrane is formed on the basal side of an epithelial cell. Examples of the components of the basement membrane include type-IV collagen, laminin, heparan sulfate proteoglycans (perlecans), entactin/nidogen, cytokines, growth factors, and the like. It can be detected through a technique such as histological staining including PAM staining and immunohistochemistry in which an antibody (an anti-laminin antibody, an anti-type-IV collagen antibody, etc.) against a constitution component of the basement membrane whether or not the basement membrane is present in a tissue derived from a living organism and the cell population produced using the production method of the present invention and the like.

The term "basement membrane preparation" refers to one containing the constituent components of the basement membrane that have a function of controlling the epithelial cell-like morphology, differentiation, proliferation, motion, functional expression, and the like when desired cells having an ability to form a basement membrane are seeded and cultured on the basement membrane preparation. In the present invention, when the adherent culture of cells is conducted, the cells can be cultured in the presence of the basement membrane preparation. Here, the term "constituent components of the basement membrane" refers to a thin membrane composed of extracellular matrix molecules that is present between the epithelial cell layer and the interstitial cell layer or the like in an animal tissue. The basement membrane preparation can be produced by, for example, removing cells that have an ability to form a basement membrane and attach to a support via the basement membrane, from the support using a solution having an ability to solubilize lipids of the cells, an alkaline solution, or the like. Examples of the basement membrane preparation include products that are commercially available as basement membrane preparations, such as Matrigel (manufactured by Corning Incorporated), Geltrex (manufactured by Thermo Fisher Scientific Inc.), and products containing extracellular matrix molecules (e.g., laminin, type-IV collagen, heparan sulfate proteoglycans, entactin, and the like) known as the basement membrane components.

Laminin is an extracellular matrix protein that is a hetero trimeric molecule composed of α, β, and γ chains and includes isoforms with subunit chains having different compositions. Specifically, laminin includes about 15 types of isoforms containing a combination of one of 5 types of α chains, one of 4 types of β chains, and one of 3 types of γ chains. The name of laminin is determined by using the numerals for the α chain (α1 to α5), the β chain (β1 to β4), and the γ chain (γ1 to γ3) in combination. For example, laminin containing a combination of the α5 chain, the β1 chain, and the γ1 chain is referred to as laminin 511. The laminin may be natural laminin, or laminin artificially synthesized using a gene recombination technology or the like, or a synthesized protein or synthesized peptide based on the laminin.

Examples of laminin or a fragment thereof used in the present invention include laminin-111 and a fragment containing the E8 region thereof, laminin-211 or a fragment containing the E8 region thereof (e.g., iMatrix-211), laminin-121 or a fragment containing the E8 region thereof, laminin-221 or a fragment containing the E8 region thereof, laminin-332 or a fragment containing the E8 region thereof, laminin-3A11 or a fragment containing the E8 region thereof, laminin-411 or a fragment containing the E8 region thereof(e.g., iMatrix-411), laminin-421 or a fragment containing the E8 region thereof, laminin-511 or a fragment containing the E8 region thereof(e.g., iMatrix-511 or iMatrix-511 silk), laminin-521 or a fragment containing the E8 region thereof, laminin-213 or a fragment containing the E8 region thereof, laminin-423 or a fragment containing the E8 region thereof, laminin-523 or a fragment containing the E8 region thereof, laminin-212/222 or a fragment containing the E8 region thereof, laminin-522 or a fragment containing the E8 region thereof, and the like.

The vitronectin may be natural vitronectin, or vitronectin artificially synthesized using a gene recombination technology or the like, or a synthesized protein or synthesized peptide based on the vitronectin. From the viewpoint of availability, examples of the commercially available product thereof include Vitronectin derived from human plasma (manufactured by Wako Pure Chemical Industries, Ltd), Synthemax (manufactured by Corning Incorporated), and Vitronectin (VTN-N) (manufactured by Gibco).

The fibronectin may be natural fibronectin, or fibronectin artificially synthesized using a gene recombination technology or the like, or a synthesized protein or synthesized peptide based on the fibronectin. From the viewpoint of availability, examples of the commercially available product thereof include Fibronectin solution derived from human plasma (manufactured by Wako Pure Chemical Industries, Ltd), and Retronectin (manufactured by Takara Bio Inc.).

The type of collagen is not particularly limited, but, for example, type-I collagen and type-IV collagen can be used. The collagen may be natural collagen, or collagen artificially synthesized using a gene recombination technology or the like, or synthesized peptide based on the collagen. From the viewpoint of availability, examples of the commercially available product thereof include Collagen I, human (manufactured by Corning Incorporated) and Collagen IV, human (manufactured by Corning Incorporated).

### (3) Adherent Culture of Step (1)

In the step (1) of the present invention, it is preferable to culture cells under feeder-free conditions and xeno-free conditions. Also, it is preferable to conduct all the steps (i.e., the steps (1) and (2)) of the production method of the present invention under feeder-free conditions and xeno-free conditions. In this specification, the term "feeder-free" means a culture medium or culturing conditions free of auxiliary cells (i.e., feeder cells) that are used to adjust culturing conditions for the cells to be cultured. Also, the term "xeno-free" means a culture medium or culturing conditions free of components derived from organisms that are different from the biological species from which cells to be cultured are derived.

The culture medium used in the step (1) (typically the step (1-1)) is not particularly limited as long as it is a culture medium (feeder-free culture medium) that enables undifferentiation-maintenance culture of pluripotent stem cells under feeder-free conditions, but the culture medium preferably contains an undifferentiation-maintenance factor in order to enable undifferentiation-maintenance culture.

The undifferentiation-maintenance factor is not particularly limited as long as it is a substance that has an effect of suppressing differentiation of pluripotent stem cells. Examples of the undifferentiation-maintenance factor for pluripotent stem cells that is widely used by those skilled in the art include FGF signaling pathway agents, TGFβ family signaling pathway agents, insulin, and the like. Specific examples of the FGF signaling pathway agents include fibroblast growth factors (e.g., bFGF, FGF4, and FGF8). Examples of the TGFβ family signaling pathway agents include TGFβ signaling pathway agents and Nodal/Activin signaling pathway agents. Examples of the TGFβ signaling pathway agents include TGFβ1 and TGFβ2. Examples of the Nodal/Activin signaling pathway agents include Nodal, Activin A, and Activin B. When pluripotent stem cells are cultured, the culture medium in the step (1) (typically the step (1-1)) preferably contains bFGF as the undifferentiation-maintenance factor.

The undifferentiation-maintenance factor used in the present invention is generally a mammalian undifferentiation-maintenance factor. Examples of the mammals include those described above. Since the undifferentiation-maintenance factor may have cross-reactivity between mammalian species, an undifferentiation-maintenance factor of any mammal may be used as long as the undifferentiated state of pluripotent stem cells to be cultured can be maintained, but it is preferable to use an undifferentiation-maintenance factor of the same mammal as that from which cells to be cultured are derived. For example, a human undifferentiation-maintenance factor (e.g., bFGF, FGF4, FGF8, EGF, Nodal, Activin A, Activin B, TGFβ1 or TGFβ2) is used for culture of human pluripotent stem cells. The undifferentiation-maintenance factor used in the present invention is preferably isolated. The term "isolated" means that the undifferentiation-maintenance factor is separated from the naturally-occurring state through an operation of removing factors other than target components or target cells. The undifferentiated maintenance factor may be added to the culture medium in advance.

The concentration of the undifferentiation-maintenance factor in the culture medium used in the step (1) (typically the step (1-1)) of the present invention is a concentration at which the undifferentiated state of pluripotent stem cells to be cultured can be maintained, and can be determined as appropriate by those skilled in the art. Specifically, when, for example, bFGF is used as the undifferentiation-maintenance factor in the absence of feeder cells, the concentration of the bFGF is typically 4 to 500 ng/ml, preferably 10 to 200 ng/ml, and more preferably 30 to 150 ng/ml.

Examples of the culture medium used in the step (1) (typically the step (1-1)) include culture media obtained by adding culture supplements such as undifferentiation-maintenance factors as described above and non-essential amino acids to basal culture media such as DMEM, Ham's F12, and D-MEM/Ham's F12, and commercially available undifferentiation-maintenance culture media such as Primate ES Cell Medium (manufactured by REPROCELL Inc.), StemFit AK02N (manufactured by Ajinomoto Co., Inc.), StemFit AK03 (manufactured by Ajinomoto Co., Inc.), mTeSR1 (manufactured by STEMCELL Technologies), TeSR-E8 (manufactured by STEMCELL Technologies), ReproNaive (manufactured by REPROCELL Inc.), ReproXF (manufactured by REPROCELL Inc.), ReproFF (manufactured by REPROCELL Inc.), ReproFF2 (manufactured by REPROCELL Inc.), NutriStem (manufactured by Biological Industries Ltd.), iSTEM (manufactured by Takara Bio Inc.), GS2-M (manufactured by Takara Bio Inc.), and hPSC Growth Medium DXF (manufactured by PromoCell). Since sera that are commonly used in cell culture media contain differentiation inducing factors as described below, the undifferentiation-maintenance culture medium for pluripotent stem cells is preferably a serum-free culture medium.

In the adherent culture (typically the step (1-1)) of the step (1) of the present invention, seeding of pluripotent stem cells on the culture vessel is not particularly limited as long as the pluripotent stem cells attach to the cell attachable regions of the culture vessel, but, for example, the cells may be seeded after being dispersed to a monodispersed state or a state close to the monodispersed state. Examples of a method for dispersing pluripotent stem cells include mechanical dispersion processing, cell dispersing liquid processing, and cytoprotectant addition processing. These types of processing may be conducted in combination.

The method for conducting the mechanical dispersion processing may be pipetting processing, or scraping operation using a scraper.

The cell dispersing liquid used in the cell dispersing liquid processing may be, for example, a solution containing any of: enzymes such as trypsin, collagenase, hyaluronidase, elastase, pronase, DNase, and papain; and chelating agents such as ethylenediaminetetraacetic acid (EDTA). A commercially available cell dispersing liquid such as TrypLE Select (manufactured by Life Technologies Corporation) or TrypLE Express (manufactured by Life Technologies Corporation) can also be used.

During the dispersion of pluripotent stem cells, the pluripotent stem cells may be treated with a cytoprotectant to suppress cell death. Examples of the cytoprotectant used for the cytoprotectant treatment include FGF signaling pathway agents, heparin, IGF signaling pathway agents, sera, and serum alternatives. In order to suppress cell death (particularly cell death of (human) pluripotent stem cells) induced through the dispersion, a Rho-associated coiled-coil kinase (ROCK) inhibitor (also referred to as a "ROCK inhibitor" in this specification) or a myosin inhibitor may be added during the dispersion. Examples of the ROCK inhibitor include (R)-(+)-trans-N-(4-pyridyl)-4-(1-aminoethyl)-cyclohexanecarboxamide·2HCl·H₂O(Y-27632), Fasudil (HA1077), H-1152, and the like. An example of the myosin inhibitor is Blebbistatin. From the viewpoint of undifferentiation maintenance, the ROCK inhibitor is preferable as the cytoprotectant. When the ROCK inhibitor is used, the final concentration in the culture medium as described below is 1 µM to 50 µM, preferably 3 µM to 20 µM, more preferably 5 µM to 15 µM, and even more preferably 8 µM to 12 µM. Typically, the ROCK inhibitor may be present in the culture medium during the step (1-1) and be removed using, for example, a method in which the culture medium is replaced with a culture medium free of the ROCK inhibitor 24 to 48 hours after the cells were seeded on the culture vessel.

When cells are seeded in the step (1) (typically the step (1-1)), the extracellular matrix as described above may be added to the culture medium as described below from the viewpoint of maintaining undifferentiation of pluripotent stem cells, and the like. As preferable extracellular matrix, laminin of the same mammal as that from which cells to be cultured are derived is preferably used from the viewpoint of achieving the xeno-free conditions. For example, human laminin (preferably laminin 511) is used for culture of human pluripotent stem cells. The amount of extracellular matrix added to the culture medium is not particularly limited as long as the extracellular matrix contributes to maintaining undifferentiation of pluripotent stem cells, and, for example, laminin 511 may be added at 0.5 to 2.0 µg/cm² per the cell attachable region of the culture vessel. The extracellular matrix may be added to the substrate in advance (precoating), or be added simultaneously with the cell seeding.

The cell seeding density on the culture vessel is not particularly limited as long as the cells can proliferate. The cell seeding density is typically 1.0×10¹ to 1.0×10⁶ cells/cm², preferably 1.0×10² cells/cm² to 1.0×10⁵ cells/cm², more preferably 1.2×10³ cells/cm² to 5.0×10⁴ cells/cm², 2.0×10³ cells/cm² to 2.5×10⁴ cells/cm², or 3.0×10³ cells/cm² to 5.0×10³ cells/cm².

The culture period in the step (1-1) is not particularly limited as long as pluripotent stem cells attach to the attachable regions of the culture vessel, but the culture period is typically 1 to 7 days, preferably 1 to 3 days, and more preferably 1 to 2 days.

The culture period in the step (1-2) is not particularly limited as long as pluripotent stem cells are cultured through expansion culture on the attachable regions of the culture vessel, but typically, it is sufficient that the cells cover about 50% to 100% of the cell attachable regions of the culture vessel (about 50% to 100% confluent). The culture period is typically 3 to 14 days, preferably 4 to 10 days, and more preferably 5 to 8 days.

Typically, the step (1-3) of the present invention is conducted after the pluripotent stem cells cover about 50% to 100% of the cell attachable regions of the culture vessel (about 50% to 100% confluent) as described above. The step (1-3) of the present invention is a step of inducing differentiation of the pluripotent stem cells after the expansion culture in the step (1-2) into a cell population containing neural stem cells (positive for PAX6). In this step, typically, the spontaneous differentiation into the cell population containing neural stem cells can be induced by conducting adherent culture using a culture medium free of undifferentiation-maintenance factors as described above. The cell population containing neural stem cells of the present invention may contain neural precursor cells. In an aspect, the step (1-3) of the present invention may be conducted using a culture medium to which the Wnt signal inhibitor and the TGFβ signal inhibitor are not added (a culture medium free of the Wnt signal inhibitor and the TGFβ signal inhibitor). In another aspect, the step (1-3) may be conducted using a culture medium to which the Wnt signal inhibitor and the TGFβ signal inhibitor are added (a culture medium containing the Wnt signal inhibitor and the TGFβ signal inhibitor).

In this specification, the term "cells" encompasses "cell population" unless otherwise stated. The cell population may be composed of one type of cell or two or more types of cells. The cell population may also be in the form of a cell aggregate such as a spheroid or organoid as described below.

In this specification, the term "cell population" refers to a cell group composed of two or more cells. The cell population may be composed of one type of cell or a plurality of types of cells. The cells contained in the cell population may float in the culture medium or attach to the culture vessel or the like. In the step (1) of the present invention, the cells contained in the cell population attach to the culture vessel or the like. The cells contained in the cell population may be single cells, or the cells of at least a portion of the cell population may attach to one another to form a cell population. In this specification, the "cell population" includes a cell aggregate that is a mass formed through aggregation of cells and containing cells attaching to one another. The cell aggregate also encompasses an embryoid body, a sphere, a spheroid, and an organoid. In this specification, the term "organoid" means a structure formed through accumulation of cells, and the organoid typically has a structure and functions similar to those of an organ in vivo. It is possible to confirm whether or not the structure is an organoid by, for example, confirming whether or not a layered structure is formed through microscopic observation, or checking the expression of a marker protein.

The Wnt signal inhibitor is not particularly limited as long as it can suppress the signaling mediated by Wnt. Examples of the Wnt signal inhibitor include IWR-1-endo (4-[(3aR,4S,7R,7aS)-1,3,3a,4,7,7a-hexahydro-1,3-dioxo-4,7-methano-2H-isoindol-2-yl]-N-8-quinolinyl-benzamide), IWP-2, XAV939, Dkk1, a Cerberus protein, a Wnt receptor inhibitor, a soluble Wnt receptor, the Wnt antibody, a casein kinase inhibitor, and a dominant-negative Wnt protein, but there is no limitation thereto.

The TGFβ signal inhibitor is not particularly limited as long as it can suppress the signaling mediated by TGFβ. Examples of the TGFβ signal inhibitor include SB431542 (4-(5-benzol[1,3]dioxol-5-yl-4-pyridin-2-yl-1H-imidazol-2-yl)-benzamide), LY-364947, SB-505, A-83-01, and the like, but there is no limitation thereto.

The concentrations of the Wnt signal inhibitor and the TGFβ signal inhibitor in the culture medium is not particularly limited and can be determined as appropriate as long as the differentiation of pluripotent stem cells into a cell population containing neural stem cells is induced. Typically, when IWR-1-endo is used as the Wnt signal inhibitor, the concentration thereof is typically 0.1 to 50 µM, and preferably 0.3 to 5 µM. When SB431542 is used as the TGFβ signal inhibitor, the concentration thereof is typically 0.1 to 100 µM, and preferably 1 to 10 µM. A combination of the Wnt signal inhibitor and the TGFβ signal inhibitor may be, for example, IWR-1-endo and SB431542.

The culture medium used in the step (1-3) is not particularly limited as long as it does not contain an undifferentiation-maintenance factor such as bFGF, but it can be prepared using, for example, a culture medium for use in animal cell culture as a basal culture medium. The basal culture medium is not particularly limited as long as it is a culture medium that can be used in animal cell culture, such as the BME culture medium, the BGJb culture medium, the CMRL 1066 culture medium, the Glasgow MEM culture medium, the Improved MEM Zinc Option culture medium, the IMDM culture medium, the Medium 199 culture medium, the Eagle MEM culture medium, the αMEM culture medium, the DMEM culture medium, the Ham's culture medium, the Ham's F-12 culture medium, the RPMI 1640 culture medium, the Fischer's culture medium, the Neurobasal culture medium, and mixed culture media thereof. The Glasgow MEM culture medium is preferably used. The culture media as described above can also be used in the step (2) of the present invention. The culture medium is preferably a serum-free culture medium from the viewpoint of not affecting the induction of differentiation of pluripotent stem cells into a cell population containing neural stem cells.

The culture medium used in the step (1-3) of the present invention may contain a serum alternative. The serum alternative can contain, for example, albumin, transferrin, a fatty acid, a collagen precursor, a trace element, 2-mercaptoethanol, or 3'-thiolglycerol, or an equivalent thereof or the like as appropriate. The serum alternative can be prepared using, for example, the method described in WO 98/30679. In order to facilitate the production method of the present invention, a commercially available serum alternative can be used. Examples of the commercially available serum alternative include KSR (knockout serum replacement) (manufactured by Invitrogen), Chemically-defined Lipid concentrated (manufactured by Gibco), and Glutamax (manufactured by Gibco). The serum alternative as described above can also be used in the step (2) of the present invention.

The culture medium may contain other additives as long as the induction of differentiation of pluripotent stem cells into a cell population containing neural stem cells is not adversely affected. Examples of the additives include insulin, iron sources (e.g., transferrin), minerals (e.g., sodium selenate), saccharides (e.g., glucose), organic acids (e.g., pyruvic acid and lactic acid), serum proteins (e.g., albumin), amino acids (e.g., L-glutamine and non-essential amino acids), reducing agents (e.g., 2-mercaptoethanol), vitamins (e.g., ascorbic acid and d-biotin), antibiotics (e.g., streptomycin, penicillin, and gentamicin), buffering agents (e.g., HEPES), and the like, but there is no limitation thereto. The additives as described above can also be used in the step (2) of the present invention.

The culture period in the step (1-3) of the present invention is not particularly limited as long as differentiation of pluripotent stem cells into a cell population containing neural stem cells (positive for PAX3) is induced, but the culture period is typically 2 to 30 days, preferably 7 to 25 days, and more preferably 12 to 18 days. After the end of the culture period in the step (1-3) of the present invention, the cell population containing neural stem cells obtained through the differentiation induction in the step (1-3) is detached from the cell attachable regions typically using a method as described below, and is then subjected to the step (2) of the present invention. In an aspect, the culture period of the step (1) (the steps (1-1) to (1-3)) is typically at least 6 days.

Other culturing conditions of the step (1) of the present invention such as the culture temperature, the CO₂ concentration, and the O₂ concentration can be determined as appropriate. The culture temperature is, for example, 30°C to 40°C, and preferably 37°C. The CO₂ concentration is, for example, 1% to 10%, and preferably about 5%. The O₂ concentration is, for example, about 20%.

### (4) Suspension Culture of Step (2)

In the step (2) of the present invention, the method for detaching the cell population containing neural stem cells formed in the step (1) is not particularly limited as long as neural organoids with desired quality are obtained, but natural detachment through cooling (e.g., 4°C), detachment using a buffer solution (e.g., PBS), a culture medium, or the like cooled to 4°C, detachment through treatment with an enzyme such as trypsin, collagenase, hyaluronidase, elastase, pronase, DNase, or papain or a chelating agent such as ethylenediaminetetraacetic acid (EDTA), and detachment through physical treatment using a scraper or the like may be used alone or in combination. A commercially available product such as TrypLE Select (manufactured by Life Technologies Corporation), TrypLE Express (manufactured by Life Technologies Corporation), Accutase (manufactured by Nacalai Tesque, Inc.), or Accumax (manufactured by Nacalai Tesque, Inc.) may also be used.

The suspension culture in the step (2) of the present invention is a step of inducing differentiation of the cell population containing neural stem cells detached as described above into desired neural organoids (e.g., brain organoids, cerebral organoids, cerebellar organoids, spinal organoids, mesencephalic organoids, choroid plexus organoids, hippocampal organoids, hypothalamic organoids, anterior pituitary organoids, and basal ganglia organoids). Regarding the induction of differentiation into desired neural organoids, conducting suspension culture of the detached cell population containing neural stem cells in a culture medium containing an appropriate differentiation inducing factor makes it possible to induce spontaneous differentiation. In this specification, the term "suspension culture" means culture conducted under conditions that maintain a state in which cells or cell aggregates float in a culture solution, namely culture conducted under conditions that prevent a firm cell-substratum junction from being formed between a cell or a cell aggregate and the culture vessel. The induction of differentiation into neural organoids as described above can be conducted by using a method known per se and determining conditions such as a differentiation inducing agent, a culture medium, and a culture period as appropriate.

A method known per se can be used for the suspension culture in the step (2) of the present invention as appropriate, and examples thereof include static culture, rotation culture, culture under a turbulent condition (environment), culture under a microgravity environment (including a gravity-free environment), and hanging-drop culture, and combinations thereof. In an aspect, the culture in the step (2) of the present invention is a combination of static culture and rotation culture. When static culture and rotation culture are combined, the static culture period is, for example, 0 to 10 days after the cell population containing neural stem cells obtained in the step (1) is detached, and preferably 0 to 4 days. In another aspect, the culture in the step (2) of the present invention is rotation culture.

A culture vessel used for suspension culture in the step (2) of the present invention is not particularly limited as long as "suspension culture" can be conducted, and those skilled in the art can select the culture vessel as appropriate. Examples of such a culture vessel include a flask, a tissue culture flask, a dish, a petri dish, a tissue culture dish, a multi-dish, a microplate, a microwell plate, a micropore, a multi-plate, a multi-well plate, a chamber slide, a laboratory dish, a tube, a tray, a culture bag, a roller bottle, and the like. Furthermore, a bioreactor is exemplified as a vessel for suspension culture. Examples of the shape of the bottom surface of the culture vessel include a U-bottom, a V-bottom, a µ-bottom, and the like. A culture vessel (e.g., EZSPHERE (AGC Techno Glass Co., Ltd.) with the bottom surface having, for example, projections and depressions, pits, and the like may be used. These culture vessels are preferably cell non-attachable vessels in order to enable suspension culture. As the cell non-attachable culture vessel, a culture vessel whose surface is not artificially treated (e.g., not coated with extracellular matrix or the like) for the purpose of improving the adhesion to the cells, and the like can be used.

In this specification, the conditions and the like of the rotation culture are not particularly limited as long as a method known per se (e.g., ML Wegscheid. et al., Cell Rep. 2021 Jul 6; 36(1): 109315.) is used to generate a swirl flow, but, for example, the rotational rate is typically 1 rpm to 200 rpm, and preferably 50 rpm to 100 rpm.

In this specification, the culture under a turbulent condition (environment) is not particularly limited as long as it is conducted in an environment in which a method known per se (e.g., ITO Y. et al., Cell 174: 636-648 e18, 2018) is used to generate a turbulent flow.

In this specification, the conditions and the like of the culture under a microgravity environment (including a gravity-free environment) are not particularly limited as long as a method known per se (e.g., WO 2019/107546) is used to conduct the culture under an environment in which the gravity is lower than that on the ground, and, for example, a microgravity cell culture apparatus (clinostat) (Yamato Scientific Co., Ltd.) and the like can be used.

In this specification, the hanging-drop culture is not particularly limited as long as a method known per se (e.g., Z Gania. et al., Future Pharmacol. 2022, 2(3), 360-376) is used to conduct the culture such that a culture medium that contains a cell population containing neural stem cells is attached to the bottom surface of a plate and the lower surface of the attached culture medium (droplet) has a hemispherical shape due to the action of gravity.

Neural organoids can be produced by, for example, culturing the cell population containing neural stem cells obtained in the step (1) of the present invention in a culture medium for inducing differentiation into a neural organoid of interest in accordance with the methods described in Sasai Y. et al., Cell Stem Cell. 12: 520-530 (2013), Sakaguchi H. et al., Nat Commun. 6: 8896 (2015), Kadoshima T. et al., Proc Natl Acad Sci U S A. 2014 May 20; 111(20): 7498, Sakaguchi H. et al., Stem Cell Reports 13 (3), 458-473, 2019, JP 2021-72818A, JP 2020-195349A, and the like. Neural organoids can be induced in a self-organizing manner by continuing the culture of the cell population containing neural stem cells under the conditions of the suspension culture in the step (2) of the present invention.

Examples of the culture medium for inducing differentiation into a neural organoid include a culture medium containing SB431542 and IWR-1-endo for inducing a brain organoid, an N2 culture medium (produced by adding an N2 supplement to a DMEM/F12 culture medium) for inducing a cerebral (cortex) organoid, a culture medium containing FGF2 and insulin for inducing a cerebellar organoid, a culture medium containing CHIR99021, bFGF, SB431542, and retinoic acid for inducing spinal organoid, a culture medium containing insulin and bFGF for inducing a mesencephalic organoid, a culture medium containing CHIR and BMP4 for inducing a choroid plexus organoid and a hippocampal organoid, a culture medium containing SAG, BMP4, and KSR (additionally FGF2) for inducing a hypothalamic organoid and a pituitary (anterior pituitary) organoid, a culture medium containing SHH for inducing a basal ganglia organoid, and the like, but there is no limitation thereto. The culture medium for inducing differentiation into a neural organoid may be changed as appropriate during the induction of an organoid.

As described above, it is possible to confirm whether or not the induced neural organoid is a neural organoid of interest by, for example, confirming whether or not a layered structure is formed through microscopic observation, or checking the expression of a marker protein. For example, in the case of the cerebral (cortex) organoid, an organoid having at least one combination of a neural stem cell (positive for PAX6), and a cortical plate (positive for CTIP2) and cortical layer (positive for CTIP2 (fifth/sixth layer), SATB2, CUX1, or the like) that are formed to surround the neural stem cell can be determined as the cerebral cortex organoid.

The step (2) of the present invention may be culture under feeder-free conditions and/or xeno-free conditions during the entire period or a part of the period. The step is preferably conducted under feeder-free conditions and xeno-free conditions during the entire period from the viewpoint of clinical use.

In another aspect, a neural organoid obtained using the production method of the present invention is also provided.

The production method (typically the step (2)) of the present invention may include step of harvesting obtained organoids of interest. The harvested organoids may be cryopreserved using a cell cryopreservation solution.

All the contents described in "(1) Step (1)", "(2) Culture Vessel and Cells", and "(3) Adherent Culture of Step (1)" are applied to the culturing method, the culture conditions, the definitions, and the like other than those described above in the step (2) of the production method of the present invention.

### 2. Transplantation Therapy Agent of the Present Invention

The present invention also provides a transplantation therapy agent containing neural organoids produced using "1. Production Method of the Present Invention" or cells included in the neural organoids. It is sufficient that a therapeutically-effective amount of the transplantation therapy agent of the present invention is administered to a subject (for example, a mammal (e.g., a human, a mouse, a rat, a monkey, a cow, a horse, a pig, or a dog) that suffers from a disease or damage caused by a disorder of the telencephalon, the diencephalon, the mesencephalon, the metencephalon, the spinal cord, and the like, and desires to be treated by cell transplantation therapy, and the therapeutically-effective amount can be changed depending on the factors such as the age, the weight, and the severity of the disease of the transplantation subject. Examples of the disease include the Parkinsons's disease, the Huntington's chorea, the Alzheimer's disease, a cerebrovascular disorder (showing a symptom of a stroke, more specifically an ischemic cerebrovascular disease (a cerebral infarction, a transient cerebral ischemic attack) or hemorrhagic encephalopathy (cerebral hemorrhage, subarachnoid hemorrhage)), epilepsy, a traumatic brain injury, a motor neuron disease, a neurodegenerative disease, and the like. Examples of the damage include damage after a brain surgery (e.g., after excision of brain tumor) and the like.

When the transplantation therapy agent is used in cell transplantation therapy, it is desirable to use cells derived from induced pluripotent stem cells established from somatic cells with the same or substantially the same HLA genotype as that of a transplantation subject, from the viewpoint of preventing rejection. Here, "substantially the same" means that the HLA genotypes are identical to an extent that the immunological reaction to the transplanted cells can be suppressed using an immunosuppressive agent, and, for example, somatic cells with the HLA type in which three genetic loci (HLA-A, HLA-B, and HLA-DR) are respectively identical, four genetic loci (HLA-C in addition to the three loci above) are respectively identical, or six genetic loci (HLA-DP and HLA-DQ in addition to the four loci above) are respectively identical are used. When a sufficient amount of cells cannot be obtained due to the age, diathesis, or the like, the cells can be embedded in a capsule made of polyethylene glycol or silicone, a porous vessel, or the like for the purpose of avoiding rejection and then transplanted.

As described above, the transplantation therapy agent of the present invention may contain cells included in neural organoids (e.g., brain organoids, cerebral organoids, cerebellar organoids, spinal organoids, mesencephalic organoids, choroid plexus organoids, hippocampal organoids, hypothalamic organoids, anterior pituitary organoids, and basal ganglia organoids) obtained using the production method of the present invention. In this specification, the "cells included in neural organoids" are not particularly limited as long as they are cells included in neural organoids obtained using the production method of the present invention, but examples thereof include neural stem cells, neural precursor cells, neurons, glial precursor cells (e.g., astrocytes, microglia, oligodendrocytes, ependymal cells, and Schwann cells), and the like. As described above, the "cells included in neural organoids" may be constituted by one cell, single cells, or a cell population.

The cells included in neural organoids that can be contained in the transplantation therapy agent of the present invention can be obtained as one cell, single cells, or a cell population by, for example, subjecting neural organoids obtained using the production method of the present invention to treatment with a cell dispersing liquid containing an enzyme or the like as described above, cutting such as physical cutting (using, for example, a scalpel, scissors, or an injection needle of 27 gauges (G) to 21 G), suction with a pipette, or the like. As described above, neural organoids obtained using the production method of the present invention have stable quality (and the quality thereof is equal among the organoids), and therefore, the cells included in the neural organoids also have stable (equal) quality, and are also suitable for the transplantation therapy agent of the present invention.

The transplantation therapy agent of the present invention can be prepared through mixing with a pharmaceutically acceptable aqueous liquid, and the like. Accordingly, in an aspect, a method for producing a transplantation therapy agent is also provided, the method including a step of formulating neural organoids produced using the production method of the present invention. This production method can also include a step of preparing the neural organoids, preserving the neural organoids, and the like.

For example, a buffer, an isotonic agent, a pH adjustor, an antioxidant, a chelating agent, and the like can be selected as appropriate and added to the pharmaceutically acceptable aqueous liquid that can be contained in the cell transplantation therapy agent of the present invention to an extent that the viability and the physiological activity of the neural organoid to be transplanted are not adversely affected.

Examples of the buffer include a phosphate buffer, a borate buffer, a citrate buffer, a tartrate buffer, an acetic buffer, an amino acid, ε-aminocaproic acid, and the like.

Examples of the isotonic agent include saccharides such as D-sorbitol, D-glucose, and D-mannitol, polyhydric alcohols such as glycerin and propylene glycol, salts such as sodium chloride, boric acid, and the like.

Examples of the chelating agent include sodium edetate, citric acid, and the like.

Examples of the pH adjustor include sodium hydroxide, potassium hydroxide, sodium carbonate, sodium hydrogen carbonate, boric acid or salts thereof (borax), hydrochloric acid, citric acid or salts thereof (sodium citrate, sodium dihydrogen citrate, and the like), phosphoric acid or salts thereof (disodium hydrogen phosphate, potassium dihydrogen phosphate, and the like), acetic acid or salts thereof (sodium acetate, ammonium acetate, and the like), tartaric acid or salts thereof (sodium tartrate and the like), and the like.

Examples of the antioxidant include ascorbic acid, glutathione, sodium hydrogen sulfite, dry sodium sulfite, sodium pyrosulfite, tocopherol, and the like.

Specific examples of the pharmaceutically acceptable aqueous liquid include aqueous solutions such as a physiological saline solution and isotonic solutions containing glucose and other adjuvants (e.g., D-sorbitol, D-mannitol, and sodium chloride).

The transplantation therapy agent of the present invention may be blended with, for example, a soothing agent (e.g., benzalkonium chloride or procaine hydrochloride), a stabilizer (e.g., human serum albumin or polyethylene glycol), a preservative, an antioxidant, and the like.

The transplantation therapy agent of the present invention can be used as follows: it is provided in a state of being cryopreserved under the conditions commonly used for cryopreservation of cells, and is thawed before use. In such a case, the cell transplantation therapy agent may further contain serum or a substitute thereof, an organic solvent (e.g., DMSO), and the like. In such a case, the concentration of the serum or the alternative thereof is not particularly limited, but can be about 1% (v/v) to about 30% (v/v) and preferably about 5% (v/v) to about 20% (v/v). The concentration of the organic solvent is not particularly limited, but can be 0% (v/v) to about 50% (v/v) and preferably about 5% (v/v) to about 20% (v/v).

Neural organoids contained in the transplantation therapy agent of the present invention may be produced using, as the raw material, pluripotent stem cells having a gene engineered through genome editing. All the contents described in "1. Production Method of the Present Invention" are applied to the matters required for the transplantation therapy agent of the present invention other than those described above.

### 3. Screening Method of the Present Invention

The present invention also provides a method for screening a therapeutic or prophylactic medicine for a neurodegenerative disease, the method including:
(1) a step of culturing a neural organoid produced using the production method of the present invention or cells included in the neural organoid in the presence or absence of a test substance;
(2) a step of evaluating a degree of neurodegeneration of the neural organoid or the cells included in the neural organoid; and
(3) a step of selecting the test substance as a candidate substance of a therapeutic or prophylactic medicine for a neurodegenerative disease when progression of the neurodegeneration of the neural organoid or the cells included in the neural organoid is more suppressed in the presence of the test substance than in the absence of the test substance in the step (2).

The degree of neurodegeneration of the neural organoids and the cells included in the neural organoids can be evaluated using, as indices, a cerebral cortex neuron marker, the form of the layer structure, electrophysiological responsivity, a metabolite, the expression of an apoptosis-related gene, and the like.

The cells included in neural organoids used in the screening method of the present invention can be obtained as one cell, single cells, or a cell population by, for example, subjecting neural organoids obtained using the production method of the present invention to treatment with a cell dispersing liquid containing an enzyme or the like as described above, cutting such as physical cutting (using, for example, a scalpel, scissors, or an injection needle of 27 gauges (G) to 21 G), suction with a pipette, or the like. As described above, neural organoids obtained using the production method of the present invention have stable quality (and the quality thereof is equal among the organoids), and therefore, the cells included in the neural organoids also have stable (equal) quality, and are also suitable for conducting the screening method of the present invention.

Examples of the test substance used in the screening method of the present invention include a cell extract, a cell culture supernatant, a microbial fermentation product, an extract derived from a marine organism, a plant extract, a purified or crude protein, a peptide, a non-peptide compound, a synthetic low-molecular-weight compound, and a natural compound. The test substance can also be obtained using any of many approaches in combinatorial library methods known in the art, including (1) a biological library, (2) a synthetic library method using deconvolution, (3) a "one-bead one compound" library method, and (4) a synthetic library method using affinity chromatography sorting. The biological library method using affinity chromatography sorting is limited to a peptide library, but four other approaches can be applied to a peptide library, a non-peptide oligomer library, or a low-molecular-weight compound library of compounds (Lam (1997) Anticancer Drug Des. 12: 145-67). Examples of a method for synthesizing a molecular library can be found in the art (DeWitt et al. (1993) Proc. Natl. Acad. Sci. USA 90: 6909-13; Erb et al. (1994) Proc. Natl. Acad. Sci. USA 91: 11422-6; Zuckermann et al. (1994) J. Med. Chem. 37: 2678-85; Cho et al. (1993) Science 261: 1303-5; Carell et al. (1994) Angew. Chem. Int. Ed. Engl. 33: 2059; Carell et al. (1994) Angew. Chem. Int. Ed. Engl. 33: 2061; Gallop et al. (1994) J. Med. Chem. 37: 1233-51). The compound library can be produced as solutions (see Houghten (1992) Bio/Techniques 13: 412-21) or beads (Lam (1991) Nature 354: 82-4), chips (Fodor (1993) Nature 364: 555-6), bacteria (U.S. Patent No. 5,223,409), spores (U.S. Patent No. 5,571,698, U.S. Patent No. 5,403,484, and U.S. Patent No. 5,223,409), plasmids (Cull et al. (1992) Proc. Natl. Acad. Sci. USA 89: 1865-9), or phages (Scott and Smith (1990) Science 249: 386-90; Devlin (1990) Science 249: 404-6; Cwirla et al. (1990) Proc. Natl. Acad. Sci. USA 87: 6378-82; Felici (1991) J. Mol. Biol. 222: 301-10; U.S. Publication No. 2002103360).

Neural organoids used in the screening method of the present invention may be produced using, as the raw material, pluripotent stem cells or disease-specific pluripotent stem cells having a gene engineered through genome editing. Examples of the disease include the Parkinsons's disease, the Huntington's chorea, the Alzheimer's disease, a cerebrovascular disorder (showing a symptom of a stroke, more specifically an ischemic cerebrovascular disease (a cerebral infarction, a transient cerebral ischemic attack) or hemorrhagic encephalopathy (cerebral hemorrhage, subarachnoid hemorrhage)), epilepsy, a traumatic brain injury, a motor neuron disease, a neurodegenerative disease, and the like.

All the contents described in "1. Production Method of the Present Invention" and "2. Transplantation Therapy Agent of the Present Invention" are applied to the matters required to conduct the screening method of the present invention other than those described above.

Hereinafter, the present invention will be described more specifically by way of examples, but the present invention is not limited to these examples by any means.

### Examples

### <Method for Producing TS-02 (Culture Vessel)>

A culture vessel having cell attachable regions was produced by covering a 35-mm diameter dish having a surface coated with a hydrophilic polymer (manufactured by Sumitomo Bakelite Co., Ltd.; product name: PrimeSurface (registered trademark)), with a metal mask with a plurality of (100) circular holes each having a diameter of 1,500 µm (manufactured by Mitani Micronics Co., Ltd.), and conducting plasma treatment (under a gas pressure of 20 Pa, a conduction current of 20 mA, and an irradiation period of 30 seconds) from above the metal mask using a plasma irradiation apparatus (manufactured by Vacuum Device Inc.; product name: Plasma Ion Bombarder PIB-20).

A temperature-responsive block copolymer to be used to coat the culture vessel was synthesized. 0.40 g (0.1 mmol) of 4-cyano-4-[(dodecylsulfanylthiocarbonyl)sulfanyl]pentanoic acid, 7.11 g (50 mmol) of n-butylmethacrylate, and 33 mg (0.2 mmol) of azobis(isobutyronitrile) were added to a test tube and were dissolved in 50 ml of 1,4-dioxane. After being degassed through nitrogen bubbling for 30 minutes, the mixture was reacted at 70°C for 24 hours. After completion of the reaction, the reaction solvent was distilled off using a rotary evaporator under reduced pressure to concentrate the reaction solution. The concentrated solution was poured into 250 ml of methanol, and a precipitated yellow oily substance was collected and dried under reduced pressure to obtain an n-butylmethacrylate polymer.

0.9 g (0.3 mmol) of the n-butylmethacrylate, 8.14 g (72 mmol) of N-isopropylacrylamide, and 5 mg (0.03 mmol) of azobis(isobutyronitrile) were added to a test tube and were dissolved in 15 ml of 1,4-dioxane. After being degassed through nitrogen bubbling for 30 minutes, the mixture was reacted at 65°C for 17 hours. After completion of the reaction, the reaction solvent was diluted with acetone, the mixture was poured into 500 ml of hexane, and a precipitated solid was collected and dried under reduced pressure. The dried solid was dissolved in acetone again and the resulting solution was poured into 500 ml of pure water. A precipitated solid was collected and dried under reduced pressure to obtain a diblock copolymer of N-isopropylacrylamide and n-butylmethacrylate.

The block copolymer was dissolved in ethanol to prepare a 0.8 wt% solution. 50 µl of this solution was dropped into the above-described culture vessel having the cell attachable regions, and spin-coating was conducted at 2000 rpm for 60 seconds. The culture vessel was dried at room temperature for 1 hour. Furthermore, the culture vessel was immersed in pure water for 24 hours for washing to produce a culture vessel (TS-02) coated with the block copolymer.

### <Measurement of Response Temperature of Block Copolymer>

A substrate coated with the block copolymer was immersed in water, and the bubble contact angle (θ) (°) was measured at an interval of 5°C within a range of 20°C to 45°C. The contact angle with water (180-θ) (°) was determined at each temperature, and the response temperature was calculated using the midpoint method. Note that θ was the contact angle of 3-µl bubble measured in water using a contact angle meter DM300 manufactured by Kyowa Interface Science Co., Ltd. The response temperature was 33°C.

### <Measurement of Thickness of Temperature-Responsive Polymer>

The thickness of the temperature-responsive polymer with which the culture vessel was coated was determined by calculating the coating amount per unit area from the surface area of the vessel, the concentration of the polymer in the solution, and the volume of the solution dropped onto the culture vessel with the specific gravity of the polymer being taken as 1. The thickness of the temperature-responsive polymer determined using this method was 29.4 nm.

### <Measurement of Composition of Temperature-Responsive Polymer>

The composition of the temperature-responsive polymer was determined through a proton nuclear magnetic resonance (¹H-NMR) spectroscopic analysis using a nuclear magnetic resonance measurement apparatus (manufactured by JEOL Ltd.; product name: JNM-GSX400), or a carbon nuclear magnetic resonance (¹³C-NMR) spectroscopic analysis using a nuclear magnetic resonance measurement apparatus (manufactured by Bruker; product name: AVANCE III HD500). The composition of the temperature-responsive polymer determined using this method was composed of 96 wt% of the N-isopropylacrylamide polymer and 4 wt% of the n-butylmethacrylate polymer.

### <Culturing Method>

Undifferentiation-maintenance culture was conducted using a StemFit (AK02) culture medium, and human pluripotent stem cells after proliferation culture were detached through enzyme treatment (TrypLE Select) and were seeded on a TS-02 substrate at a cell density of 14,000 cells/cm² or a cell density of 15,000 cells/cm² (Day 0 of the culture). When the cells were seeded, iMatrix (1.5 µg/cm²) and a ROCK inhibitor Y-27632 (10 µM) were added to maintain undifferentiation of induced pluripotent stem cells. The proliferation culture of the cells that had attached onto the TS-02 substrate was conducted over 5 to 7 days, and the culture medium was changed to a differentiation inducing culture medium when the cells became confluent. As the differentiation inducing culture medium, a conditioned culture medium was used that was prepared by adding NEAA (1%), sodium pyruvate (1%), and 2-mercaptoethanol to a GMEM culture medium serving as a basal culture medium. In addition, KSR (20%) was added to the differentiation culture medium in order to promote neurodifferentiation, and then the differentiation was induced for 16 to 20 days. Thereafter, PBS or a culture medium cooled to 4°C was used to cause spontaneous detachment of the cells (a cell population containing neural stem cells) that attached onto the TS-02.

As a differentiation inducing culture medium for the suspension culture of the detached cell fragments, a conditioned culture medium was used that was prepared by adding GlutaMAX, CD Lipid concentrate (1%), penicillin/streptomycin (1%), amphotericin B (0.1%), and N2-supplement (1%) to the DMEM/F12 culture medium serving as a basal culture medium. The cells were transferred onto a 90-mm dish (MS-1390R (manufactured by Sumitomo Bakelite Co., Ltd.)), and static culture was conducted for 4 days. Then, the culture was changed to rotation culture, and the rotation culture was conducted for 13 days or longer (FIG. 1). Note that the culture medium was replaced once every 1 to 3 days.

### Example 1: Induction of Cerebral Cortex Organoids

In accordance with the method illustrated in FIG. 1, adherent culture of human iPS cells (201B7) on TS-02 (spot diameter: 1,500 µm) was conducted for 20 days, and then rotation culture was conducted for 6 days. As a result, a tissue structure could be confirmed under a microscope. By conducting the culture for another 9 days, a plurality of tissue structures could be confirmed more clearly. The cells at this time (Day 35) were fixed, and sections thereof were prepared and immunostained. Thus, neural stem cells (positive for PAX6) and cortical plates (positive for CTIP2) formed to surround the neural stem cells could be confirmed, and it was found that cerebral cortex organoids could be induced (FIG. 2).

### Example 2: Verification of Induction Efficiency through Examination on Differentiation Inducing Signals

It was found that the formation of the layer structure of the cerebral cortex organoid was also observed by adding an ectoderm inducing factor SB431542 (TGFβ signal inhibitor) (5 µM) and a Wnt antagonist IWR-1-endo (Wnt signal inhibitor) (3 µM) from Day 0 to Day 18 of the differentiation induction in the differentiation induction of Example 1 above (FIG. 3).

### Example 3: Comparison of Induction Efficiency with 96-well Plate

In the differentiation induction of Example 2 above, the efficiencies of induction of cerebral cortex organoids on a 96-well plate and the TS-02 were compared. Three types of human iPS cells (Ff-I01s04, Ff-I14s03, Ff-WJs524) were used. On the 96-well plate, a cerebral cortex layer structure could be confirmed in a portion of Ff-I01s04 on Day 35 of the differentiation induction. Meanwhile, the cerebral cortex layer structure could not be confirmed in Ff-I14s03 even though the culture was continued for 35 days. In the case of Ff-WJs524, the cells started to die on Day 18 of the differentiation induction, and thus the experiment was stopped (FIG. 4). On the TS-02, survival of all the cells was confirmed on Day 35 of differentiation induction, and the formation of the layer structure of a cerebral cortex organoid was observed thereinside. Neural stem cells (positive for PAX6) and cortical plates (positive for CTIP2) formed to surround the neural stem cells could be confirmed through immunostaining, and it was found that cerebral cortex organoids could be induced (FIG. 5). It was found from the results above that cerebral cortex organoids can be efficiently induced from human pluripotent stem cells using this differentiation inducing method.

### Example 4: Efficiency of Induction of Cerebral Cortex Organoids Depending on Micropatterning Area

In the differentiation induction of human iPS cells (201B7) in Example 2 above, it was verified whether the induction efficiency was affected, by comparing 5 types of micropatterning areas of the TS-02 whose diameters were 150 µm, 200 µm, 500 µm, 750 µm, and 1500 µm. As a result, on Day 25 of the differentiation induction, the formation of the cerebral cortex organoid layer structure was observed under all the conditions, and it was thus found that the differentiation induction efficiency is less likely to extremely decrease depending on the area (FIG. 6).

### Example 5: Formation of Cerebral Cortex Organoids Depending on Difference in Timing of Detaching Cells from TS-02

In the differentiation induction of Example 2 above, it was examined how the efficiency of induction of differentiation into cerebral cortex organoids changed by setting the timing of transition to rotation culture to Day 6, Day 12, and Day 18 of the differentiation induction. Two types of human iPS cells (Ff-I01s04, Ff-I14s03) were used. On Day 49 of the differentiation induction, the tissue structure could be confirmed under a microscope under all the conditions. The cerebral cortex organoids were efficiently induced when the transition to rotation culture was conducted on Day 12 and Day 18 of the differentiation induction, whereas the induction efficiency was poor when the transition to rotation culture was conducted on Day 6 of the differentiation induction (FIG. 7).

### Example 6: Equalization of Size of Cerebral Cortex Organoids Depending on Conditions of Rotation Culture

Human iPS cells (201B7) were cultured for 18 days in the differentiation induction of Example 2 above, and cerebral cortex organoids were induced under the conditions that rotation culture was conducted using a 90-mm dish after the detachment of the cells, or the conditions that physical stimulation was likely to be applied during rotation due to projections and depressions being formed on a dish (EZSPHERE (AGC Techno Glass Co., Ltd.)). It was found from the results that after the rotation culture for 17 days, the shapes of the organoids varied when the culture was conducted using the 90-mm dish, whereas the sizes were equalized as a whole when the culture was conducted using the EZSPHERE (FIG. 8). Furthermore, when differentiation of human ES cells (Kh-1) was induced in the same manner, it was found that a uniform cerebral cortex layer structure was induced as the internal structure on Day 35 (FIG. 9).

### Example 7: Variation of Marker Expression in Cerebral Cortex Organoids

Human iPS cells (201B7) were cultured for 35 days in the differentiation induction of Example 2 above, and variation of the expression of markers for PAX6 and CTIP2 between organoids was verified. Cerebral cortex organoids on Day 35 of the differentiation induction were fixed and were stained with PAX6 and CTIP2, and 18 organoids were evaluated using a cell sorter. It was found from the results that the variation of PAX6 expression was 27.0±12.7%, and the variation of CTIP2 expression was 37.7±10.9% (FIG. 10).

### Example 8: Induction of Cerebral Cortex Organoids

In Example 1, the cells were cultured on the StemFit AK02 until they became confluent, and then the culture medium was changed to the differentiation inducing culture medium (GMEM culture medium) without conducting reseeding. This method was slightly modified, and as shown in FIG. 11, when the culture medium was changed to the differentiation inducing culture medium, the cells were reseeded and were then cultured in the same manner as in Example 1. The cells were cultured in the presence of Y-27632 for 3 days after the reseeding. As a result, similarly to Example 1, cerebral cortex organoids having a neuroepithelial structure were obtained (FIGS. 12 and 13). It was shown that in these cerebral cortex organoids, structures that were highly uniform among the organoids were formed compared with the conventional method using a 96-well plate (FIG. 15).

### Industrial Applicability

The present invention is useful because with the present invention, it is possible to provide a method for producing a neural organoid using a culture vessel that has a plurality of independent cell attachable regions and is configured to have cells that will attach to the regions to be present in the same culture medium, and the method can be used to produce a neural organoid with stable quality in a large amount at low cost. In particular, since the cell attachable regions are determined in advance in the culture vessel used in the present invention, it is possible to prevent, for example, the adjacent cells from attaching to each other due to the operation such as replacement of the culture medium. Therefore, it is possible to produce neural organoids with stable quality in a large amount through a single operation. Also, the production method of the present invention is also useful because the neural organoid produced using the production method of the present invention has stable (equal) quality, and therefore, it is expected that a mechanism of a disease (particularly a neurodegenerative disease) that has been difficult to clarify hitherto will be clarified, and drug development research, regenerative medicine, and the like will be developed, by using the transplantation therapy agent containing the neural organoid, or the method for screening a therapeutic or prophylactic medicine for a neurodegenerative disease using the neural organoid.

The present application claims the benefit of priority based on Japanese Patent Application No. 2023-044570 filed in Japan (filing date: March 20, 2023), which is incorporated herein by reference in its entirety.

## Claims

1. A method for producing a neural organoid, comprising:
(1) a step of forming a cell population containing neural stem cells through adherent culture of pluripotent stem cells in a culture vessel that has a plurality of independent cell attachable regions and is configured to have cells that will attach to the regions to be present in the same culture medium; and
(2) a step of detaching the cell population containing neural stem cells formed in the step (1) and conducting suspension culture of the cell population.

2. The method according to claim 1, wherein the suspension culture of the step (2) includes a step of conducting rotation culture.

3. The method according to claim 1 or 2, wherein the neural organoid is selected from the group consisting of a brain organoid, a cerebral organoid, a cerebellar organoid, a spinal organoid, a mesencephalic organoid, a choroid plexus organoid, a hippocampal organoid, a hypothalamic organoid, an anterior pituitary organoid, and a basal ganglia organoid.

4. The method according to any one of claims 1 to 3, wherein the neural organoid is a cerebral organoid.

5. The method according to any one of claims 1 to 4, wherein the step (1) is conducted for at least 6 days.

6. The method according to any one of claims 1 to 5, wherein the cell attachable regions contain extracellular matrix.

7. The method according to any one of claims 1 to 6, wherein the cell attachable regions contain a temperature-responsive polymer.

8. The method according to any one of claims 1 to 7, wherein the number of the cell attachable regions is five or more.

9. The method according to any one of claims 1 to 8, wherein the pluripotent stem cells are induced pluripotent stem cells.

10. The method according to claim 9, wherein the induced pluripotent stem cells are derived from a patient suffering from a neurodegenerative disease.

11. A neural organoid produced using the method according to any one of claims 1 to 10.

12. A transplantation therapy agent comprising the neural organoid according to claim 11 or cells included in the neural organoid.

13. A method for screening a therapeutic or prophylactic medicine for a neurodegenerative disease, comprising:
(1) a step of culturing the neural organoid according to claim 11 or cells included in the neural organoid in the presence or absence of a test substance;
(2) a step of evaluating a degree of neurodegeneration of the neural organoid or the cells included in the neural organoid; and
(3) a step of selecting the test substance as a candidate substance of a therapeutic or prophylactic medicine for a neurodegenerative disease when progression of the neurodegeneration of the neural organoid or the cells included in the neural organoid is more suppressed in the presence of the test substance than in the absence of the test substance in the step (2).
